(19) Europäisches Patentamt
European Patent Office
Office européen des brevets

(11) EP 3 102 292 B1

(12) EUROPÄISCHE PATENTSCHRIFT

(45) Veröffentlichungstag und Bekanntmachung des Hinweises auf die Patenterteilung:
**11.04.2018 Patentblatt 2018/15**

(21) Anmeldenummer: **15704211.0**

(22) Anmeldetag: **05.02.2015**

(51) Int Cl.:
***A61Q 5/02*** $^{(2006.01)}$ ***A61Q 5/12*** $^{(2006.01)}$
***A61K 8/92*** $^{(2006.01)}$

(86) Internationale Anmeldenummer:
**PCT/EP2015/000231**

(87) Internationale Veröffentlichungsnummer:
**WO 2015/117757 (13.08.2015 Gazette 2015/32)**

# (54) KOMBINATIONEN VON KATIONISCHEN HAARBEHANDLUNGSMITTELN MIT DIMETHYLAMINOPROPYLAMIDEN VON OXIDATEN NATÜRLICHER WACHSE UND DEREN VERWENDUNG IN KOSMETISCHEN ZUBEREITUNGEN, INSBESONDERE HAARPFLEGEMITTELN

COMBINATIONS OF CATIONIC HAIR TREATMENT PRODUCTS WITH DIMETHYLAMINOPROPYLAMIDES OF OXIDATION PRODUCTS OF NATURAL WAXES AND USE THEREOF IN COSMETIC COMPOSITIONS, IN PARTICULAR HAIR CARE PRODUCTS

COMBINAISON D'AGENTS DE TRAITEMENT CAPILLAIRE CATIONIQUES AVEC DES DIMÉTHYLAMINOPROPYLAMIDES DE PRODUITS D'OXYDATION DE CIRE NATURELLE ET LEUR UTILISATION DANS DES PRÉPARATIONS COSMÉTIQUES, NOTAMMENT DES PRODUITS DE SOINS CAPILLAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.02.2014 DE 102014001709**

(43) Veröffentlichungstag der Anmeldung:
**14.12.2016 Patentblatt 2016/50**

(73) Patentinhaber: **Clariant International Ltd**
**4132 Muttenz (CH)**

(72) Erfinder:
- **KITA-TOKARCZYK, Katarzyna Elzbieta**
**65812 Bad Soden (DE)**
- **NEUHOFF, Henrike**
**65193 Wiesbaden (DE)**

(74) Vertreter: **Holmes, Rosalind**
**Clariant Produkte (Deutschland) GmbH**
**Patent & License Management Chemicals**
**Industriepark Höchst, G 860**
**65926 Frankfurt am Main (DE)**

(56) Entgegenhaltungen:
**EP-A1- 1 586 298**
**EP-A1- 1 870 080**
**EP-A2- 1 435 363**
**WO-A1-2014/131514**
**WO-A2-02/102334**
**DE-A1- 10 231 886**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 102 292 B1

## Beschreibung

Die Erfindung betrifft Kombinationen zur Behandlung keratinischer Strukturen auf Basis von Dimethylaminopropylamiden von Oxidaten natürlicher Wachse kationischen Tensiden und/oder kationische Polymeren sowie deren Verwendung in kosmetischen Zubereitungen, insbesondere in Haarpflegemitteln zur Behandlung des Haars von Mensch oder Tier.

Pflegemittel der Haare wie Spülungen (Konditionierer) werden nach oder in Verbindung mit Haarshampoo eingesetzt, um nach der Haarwäsche eine bessere Kämmbarkeit von nassem und trockenem Haar zu bewirken, die statische Aufladung zu reduzieren, den Griff zu verbessern sowie den Glanz und Farbschutz der Haare zu gewährleisten. Neben einer guten Hautverträglichkeit sollen solche Stoffe aus ökologischen Gründen eine gute Bioabbaubarkeit aufweisen. Weitere Haarpflegemittel sind so genannte Masken, die auch als "deep conditioning treatments" bezeichnet werden. Dabei handelt es sich um reichhaltige, zum nach dem Einwirken lassen auszuspülende Haarkonditioniermittel, die oft cremig anmuten und eine deutlich höhere Viskosität aufweisen, als normale Haarkonditioniermittel; sich weisen meist eine gegenüber diesen deutlich erhöhte Viskosität auf. Nicht unerwähnt sollen Shampoos bleiben; deren Hauptverwendung besteht zwar im Reinigen von Haaren, zeitgemäße Shampoos sind allerdings nicht ohne zusätzliche haarkonditionierende Wirkung denkbar.

Es ist bekannt, dass bestimmte Amidamine zur Verwendung in Haarpflegemitteln geeignet sind (siehe zum Beispiel WO 02/102334, EP-A 1 586 298, EP-A 1 870 080 und die darin zitierte Literatur). In der EP-A 1 586 298 sind Haarpflegemittel offenbart, die neben einem langkettigen Alkohol Dimethylaminopropylamide enthalten, deren Säurereste zu mindestens 80 Gew.-% aus $C_{\geq 20}$ Resten bestehen, wobei $C_{22}$ aliphatische Säurereste 70 bis 95 Gew.-% ausmachen.

Obwohl mit solchen Verbindungen bereits gute Ergebnisse erzielt werden, besteht doch ein breiter Raum für Verbesserungen, beispielsweise was Konditionierleistung und Formulierbarkeit aber auch ökonomische und ökologische Aspekte der Herstellung und Benutzung angeht.

Es hat sich nun für den Fachmann überraschend und nicht vorhersehbar herausgestellt, dass Kombinationen aus kationischen Tensiden und/oder kationischen Polymeren mit Amidaminen der Formel (I),

$$R\text{-}C(O)\text{-}NH\text{-}(CH_2)_3\text{-}N(CH_3)_2 \qquad (I)$$

worin R-C(O) für die aliphatischen Säurereste eines Naturwachsoxidats steht, den Nachteilen des Standes der Technik abhelfen. Solche Kombinationen mit Dimethylaminopropylamiden von Naturwachsoxidaten entsprechen in besonderer Weise diesen neuen Anforderungen und zum Einsatz in kosmetischen Zubereitungen, insbesondere Haarpflegemitteln.

Dimethylpropylamide von längerkettigen Fettsäuren wie Montansäure ($C_{28}$) sind in der EP-A 1 435 363 als Katalysatoren für die Polyurethanherstellung offenbart. Naturwachsoxidate als Ausgangsverbindungen sind in diesem Dokument jedoch nicht beschrieben.

Eine weitere, zweite Ausführungsform der Erfindung betrifft Kombinationen aus kationischen Tensiden und/oder kationischen Polymeren mit a) 10 bis 100 Gew.-% Amid-aminen der Formel (I) und b) 90 bis 0 Gew.-% Amidaminen der Formel (II),

$$R^1\text{-}C(O)\text{-}NH\text{-}(CH_2)_3\text{-}N(CH_3)_2 \qquad (II)$$

worin $R^1$-C(O) für mindestens einen ($C_{18}$-$C_{22}$)-aliphatischen Säurerest steht. Eine dritte Ausführungsform der Erfindung betrifft Kombinationen aus kationischen Tensiden und/oder kationischen Polymeren mit Amidaminen der Formel (Ia),

$$R^2\text{-}C(O)\text{-}NH\text{-}(CH_2)_3\text{-}N(CH_3)_2 \qquad (Ia)$$

worin $R^2$-C(O) für aliphatische Säurereste steht, erhältlich durch Umsetzung von 3-(Dimethylamino)propylamin mit a) 10 bis 100 Gew.-% eines Naturwachsoxidats und b) 0 bis 90 Gew.-% mindestens einer ($C_{18}$-$C_{22}$)-aliphatischen Carbonsäure. Der Einsatz von erfindungsgemäßen Kombinationen in Haarpflegemitteln führt zu Produkten, die exzellente Konditioniereigenschaften aufweisen, gut zu verarbeiten sind und eine gute Bioabbaubarkeit zeigen. Mit den erfindungsgemäßen Amidamin Zusammensetzungen werden zudem nachhaltige und umweltverträgliche Ausgangsstoffe für die Kosmetikindustrie bereitgestellt.

Der Begriff "Naturwachs" im Sinne der Erfindung bedeutet ein natürlich vorkommendes Wachs von nachwachsendem (z. B. Reiskleiewachs) oder fossilem (z. B. Montanwachs) Ursprung.

Der Begriff "Naturwachsoxidat" bedeutet im Sinne der Erfindung, dass die nativen Wachsester zu einem Teil, bevorzugt mindestens 30 %, besonders bevorzugt mindestens 40 %, ganz besonders bevorzugt mindestens 60 % zu den entsprechenden Carbonsäuren oxidiert wurden.

Die Oxidation von fossilen sowie von nichtfossilen Naturwachsen mit Chromschwefelsäure ist seit Anfang des

20. Jahrhunderts bekannt und wird technisch anhand von fossilen Montanwachsen seit 1927 beispielsweise im heute noch betriebenen "Gersthofener Verfahren" durchgeführt. Neben dem fossilen Montanwachs lassen sich mit Hilfe dieser chromsäurebasierten Verfahren auch nachwachsende Naturwachse wie Carnaubawachs und Candelillawachs oxidieren.

Die Chromsäurebleichung genannter Naturwachse führt, in Abhängigkeit von der eingesetzten Chromsäuremenge im Verhältnis zum eingesetzten Wachs, zu hohen und definierten Säurezahlen. Bei der Oxidation mit Chromsäure kommt es im Wesentlichen zu einer Spaltung der nativen Wachsester sowie einer in-situ-Oxidation der entstandenen Wachsalkohole zu Wachssäuren. Die Höhe der Säurezahl ist ein Maß für den Gehalt an freien Wachssäuren. Die typischen Umsätze solcher Oxidationen liegen dabei im Bereich von ca. 30 bis 90 % bezüglich der Estergruppen. Die Säurezahlen liegen in Abhängigkeit vom Umsatz und Art des Naturwachses zwischen 70 und 200 mg KOH/g. Die auf diese Weise oxidierten Naturwachse besitzen daher zusätzlich zur gewünschten Aufhellung eine höhere Verseifungszahl und Säurezahl, die für Veresterungs- und Amidierungsreaktionen zugänglich sind.

Da Säure- und Alkoholkomponente meist unterschiedliche mittlere Kettenlängen aufweisen (die Alkoholkomponente ist typischerweise länger), unterscheiden sich die Naturwachsoxidate in ihrer Zusammensetzung signifikant von den Säuremischungen, die aus einer Verseifung des Wachses hervorgeht.

Solche Zubereitungen sind zum Beispiel durch Mischen entsprechender Amidamine (I) und (II) erhältlich oder, vorzugsweise, durch Umsetzung von 3-(Dimethylamino)propylamin mit

(a') 10 bis 100 Gew.-% eines Naturwachsoxidats und
(a") bis zu 90 Gew.-% mindestens einer ($C_{18}$-$C_{22}$)-aliphatischen Carbonsäure.

Natürlich ist es auch möglich, Säuren oder entsprechende Ester der Komponente (a") dem Naturwachs vor der Oxidation zuzusetzen.

Bevorzugte Mischungsverhältnisse sind im Rahmen der erfindungsgemäßen kosmetischen Zubereitungen aufgeführt.

In einer bevorzugten Ausführungsform der Erfindung enthält die erfindungsgemäße kosmetische Zubereitung wie bereits dargestellt neben den Amidaminen der Formel (I) (Komponente (a1)) eine Komponente (a2), die aus Amidaminen der Formel (II) besteht,

$$R^1\text{-C(O)-NH-C(CH}_2)_3\text{-N(CH}_3)_2 \qquad \text{(II),}$$

wobei $R^1$ ein aliphatischer Kohlenwasserstoffrest, eine geradkettige Alkylgruppe mit 10 bis 22, bevorzugt 18 bis 22, C-Atomen ist.

Besonders bevorzugt ist $R^1$ im Wesentlichen $C_{18}$- oder $C_{22}$- oder eine Mischung aus $C_{18}$ und $C_{22}$ Alkyl, insbesondere geradkettiges Alkyl.

Solche Verbindungen sind beispielsweise als Genamin® SPA und Genamin® BAP von Clariant oder als Amidet APA-22 von Kao erhältlich.

Bevorzugt sind Mischungen der Komponenten (a1) : (a2) im Gewichtsverhältnis 1 : 0,5 bis 9, besonders bevorzugt 1 : 1 bis 4, insbesondere 1 : 2 bis 3.

Überraschenderweise werden die erfindungsgemäßen Vorteile wie gute Konditionierleistungen auch von Mischungen verwirklicht, die einen Überschuss an der Komponente (a2) aufweisen. Mischungen aus (a1) und (a2) zeigen zudem besonders vorteilhafte Verarbeitungseigenschaften wie einen niedrigen Schmelzpunkt.

In einer weiteren bevorzugten Ausführungsform enthält die erfindungsgemäße Zubereitung neben den Komponenten (a1) und gegebenenfalls (a2) einen aliphatischen Alkohol mit der Formel (III) als Komponente (b) (nachfolgend als aliphatischer Alkohol (III)) bezeichnet,

$$R^3\text{-OH} \qquad \text{(III),}$$

worin $R^3$ eine aliphatische gesättigte oder ungesättigte $C_8$-$C_{30}$-Kohlenwasserstoffgruppe ist, worin lineare aliphatische Kohlenwasserstoffgruppen 80 Gew.-% oder mehr ausmachen.

Im aliphatischen Alkohol (III) als Komponente (b) gemäß dieser Erfindung bedeutet $R^3$ eine gesättigte oder ungesättigte aliphatische $C_{8-30}$-Kohlenwasserstoffgruppe, worin lineare aliphatische Kohlenwasserstoffgruppen 80 Gew.-% oder mehr, bevorzugt 85 Gew.-% oder mehr, noch mehr bevorzugt 90 Gew.-% oder mehr ausmachen. Die lineare aliphatische Kohlenwasserstoffgruppe ist bevorzugt eine lineare $C_{8-30}$-Alkyl- oder Alkenylgruppe, mehr bevorzugt eine lineare $C_{10-26}$-Alkylgruppe. Der aliphatische Alkohol (III) umfasst beispielsweise Cetylalkohol, Stearylalkohol und Behenylalkohol. Bevorzugt sind Cetylalkohol und/oder Stearylalkohol.

Wenn der aliphatische Alkohol (III) mit einem entsprechenden Grad an linearen Ketten in Kombination mit dem Amidamin (I) als Komponente (a) in dieser Erfindung verwendet wird, entfalten die resultierenden Haarpflegemittel eine

bevorzugte Viskosität, Glätte und nasses Gefühl während der Anfeuchtung.

Zum Erhalt eines ausgezeichneten Haargefühls und im Hinblick auf die Stabilität des Produktes ist der Gehalt der Komponente (a) in Haarpflegemitteln gemäß der Erfindung bevorzugt 0,1 bis 15 Gew.-%, mehr bevorzugt 0,5 bis 10 Gew.-%, noch mehr bevorzugt 0,5 bis 5 Gew.-%. Der Gehalt der Komponente (b) ist bevorzugt 0,5 bis 15 Gew.-%, mehr bevorzugt 1 bis 10 Gew.-%.

Bei all dem ist es bevorzugt, wenn die Säurezahl des Naturwachsoxidats oder der Mischung von Naturwachsoxidat und ($C_{18}$-$C_{22}$)-aliphatischen Carbonsäuren gemessen nach DIN EN ISO 2114 mindestens 70 mg KOH/g ist. Bei letzterem ist es besonders bevorzugt, wenn die Säurezahl im Bereich von 70 bis 200 mg KOH/g liegt. Bevorzugt ist es weiter, wenn das Naturwachsoxidat gewählt ist aus der Gruppe bestehend aus Reiskleiewachsoxidaten, Montanwachsoxidaten, Carnaubawachsoxidaten, Zuckerrohrwachsoxidaten, Sonnenblumenwachsoxidaten, Candellilawachsoxidaten und Jojobaöloxidaten, ganz besonders bevorzugt wird das Naturwachsoxidat gewählt unter Reiskleiewachsoxidaten und Montanwachsoxidaten. Bei all dem ist es besonders bevorzugt, wenn in dem Naturwachs, aus dem das Naturwachsoxidat hergestellt wurde, mindestens 30 % Gew.-% der nativen Wachsester zu Carbonsäuren oxidiert wurde, ganz besonders bevorzugt ist es, wenn mindestens 60 % der nativen Wachsester zu Carbonsäuren oxidiert wurden. Bei all dem ist es bevorzugt, wenn der Anteil der Amidamine mit einem Säurerest, der mindestens 24 C-Atome aufweist, mindestens 9,5 Gew.-% beträgt, besonders bevorzugt ist es, wenn der Anteil der Amidamine mit einem Säurerest, der mindestens 24 C-Atome aufweist, mindestens 25 Gew.-% beträgt, ganz besonders bevorzugt ist es, wenn dabei die Säurereste 24 bis 40 C-Atome aufweisen. Ein Verfahren zur Herstellung von Amidaminen der Formel (I), umfasst den Schritt der Umsetzung eines Naturwachsoxidats mit einer Säurezahl größer 70 mit 3-(Dimethylamino)propylamin (DMAPA).

Weiterhin bevorzugt beträgt der Anteil der Amidamine mit einem Säurerest, der mindestens 26 C-Atome aufweist, mindestens 4,5 Gew.-%, bevorzugt mindestens 10 Gew.-%, wobei die Säurereste bevorzugt 26 bis 40 C-Atome aufweisen.

Erfindungsgemäß eingesetzte Naturwachsoxidate weisen eine mittlere Säurezahl größer 70, bevorzugt größer 100, besonders bevorzugt größer 120 auf. Die Obergrenze der Säurezahl liegt im Allgemeinen bei 200.

Die Säurezahl gibt die Anzahl der mg KOH an, die zur Neutralisation von 1 g des Naturwachsoxidats verbraucht wird. Sie wird erfindungsgemäß nach DIN EN ISO 2114 bestimmt.

Neben der angegebenen Säurezahl weisen die erfindungsgemäß eingesetzten Naturwachsoxidate typischerweise eine Verseifungszahl von 110 bis 200 (gemessen nach DIN EN ISO 2114) und einen Tropfpunkt von 70 bis 100 °C (gemessen nach DIN ISO 2176) auf.

Die Herstellung der erfindungsgemäß eingesetzten Naturwachsoxidate erfolgt nach bekannten, dem Fachmann geläufigen Methoden, insbesondere durch Oxidation mit Chromschwefelsäure, wie sie in der DE-A 102 31 886 am Beispiel von Carnaubawachsen und weiter unten am Beispiel von Reiskleiewachsen beschrieben ist.

In einer bevorzugt Ausführungsform der Erfindung sind die Ausgangsverbindungen für die Amidamine der Formel (I) Montanwachsoxidate mit einer Säurezahl gemessen nach DIN ISO 2114 größer 70 mg KOH/g, bevorzugt größer 100 mg KOH/g, besonders bevorzugt größer 140 mg KOH/g.

Die erfindungsgemäßen eingesetzten Montanwachsoxidate umfassen freie aliphatische Carbonsäuren $C_{10}$ bis $C_{36}$ mit einer für Montanwachsoxidate charakteristischen Kettenlängenverteilung (siehe Abbildung 1) sowie aus Dicarbonsäuren $C_{10}$ bis $C_{36}$ zusammen. Die Monocarbonsäuren zeichnen sich durch einen hohen Anteil von Montansäure ($C_{28}$), Cerotinsäure ($C_{26}$) und Melissinsäure ($C_{30}$) in charakteristischen Verhältnissen aus. Die Kettenlängenverteilung sowie die charakteristischen Gewichtsverhältnisse können mittels GC bestimmt werden.

In Abhängigkeit vom Umsetzungsgrad der Ester enthalten die erfindungsgemäß eingesetzten Montanwachsoxidate native Ester ($C_{46}$-$C_{62}$) in Mengen kleiner 60 Gew.-%, bevorzugt kleiner 50 Gew.-%, besonders bevorzugt kleiner 40 Gew.-%. Ferner enthalten die erfindungsgemäß eingesetzten Montanwachsoxidate kleine Mengen an aliphatische Disäuren ($C_{10}$ bis $C_{32}$), in Abhängigkeit vom Umsetzungsgrad von 5 bis 20 Gew.-%. Daneben können die erfindungsgemäß eingesetzten Montanwachsoxidate auch kleine Mengen aliphatischer Alkanole ($C_{24}$ bis $C_{36}$) enthalten.

Weitere nicht näher spezifizierte Bestandteile können in geringen Konzentrationen von bis zu 5 Gew.-% auftreten.

Bevorzugt zeichnen sich die erfindungsgemäßen Montanwachsoxidate durch einen Tropfpunkt gemessen nach DIN ISO 2176 zwischen 70 °C und 90 °C, bevorzugt zwischen 75 °C und 85 °C aus.

In einer Ausführungsform der Erfindung werden die erfindungsgemäß eingesetzten Montanwachsoxidate zweistufig durch Verseifung unter Druck, optional anschließender Neutralisation und darauffolgender Oxidation mit Chromschwefelsäure hergestellt. Die Verseifung unter Druck verläuft bei 1 bis 30 bar, bevorzugt bei 5 bis 20 bar, besonders bevorzugt bei 15 bis 20 bar und erhöhter Temperatur bei 80 bis 250 °C , bevorzugt bei 180 °C bis 225 °C mit 0,5. bis 1,5-fachen Überschuss an NaOH. Weitere Herstellprozesse sind etwa in Ullmann's Encyclopedia of Industrial Chemistry, 2000, Waxes sowie in

DE 10231886 sowie in Vladimir Vcelák, Chemie und Technologie des Montanwachses, 1959, Teil B: Veredelung des Montanwachses, S. 458 ff beschrieben.

In einer weiteren bevorzugten Ausführungsform der Erfindung sind die Naturwachsoxidate Reiskleiewachsoxidate mit einer Säurezahl gemessen nach DIN ISO 2114 größer 70 mg KOH/g, bevorzugt größer 100 mg KOH/g, besonders bevorzugt größer 140 mg KOH/g.

Die erfindungsgemäß eingesetzten Reiskleiewachsoxidate umfassen freie aliphatische Carbonsäuren $C_{16}$ bis $C_{36}$ mit einer für Reiskleiewachsoxidate charakteristischen Kettenlängenverteilung. Diese zeichnet sich aus durch eine ausgeprägte Majorität von Lignocerinsäure ($C_{24}$) und deutlichen Anteilen an Behensäure ($C_{22}$) und Melissinsäure ($C_{30}$) in charakteristischen Verhältnissen. Bevorzugt liegt die darin am häufigsten vorkommende freie Carbonsäure, Lignocerinsäure ($C_{24}$-Säure) zu mindestens 10 Gew.-%, bevorzugt zu mindestens 15 Gew.-%, besonders bevorzugt zu mindestens 20 Gew.-% vor. Erfindungsgemäß liegt das charakteristische Kettenlängenverhältnis von Behensäure zu Lignocerinsäure $C_{22}$:$C_{24}$ für die Reiskleiewachsoxidate bei 1,0 : 2,0 bis 1 : 2.8, bevorzugt bei 1,0 : 2,1 bis 1,0 : 2,4. Das charakteristische Kettenlängenverhältnis von Behensäure zu Melissinsäure $C_{22}$:$C_{30}$ liegt bei den erfindungsgemäßen Reiskleiewachsoxidaten zwischen 1,0 : 0,8 bis 1 : 1,2, bevorzugt zwischen 1, 0 : 0,8 bis 1,0 : 1,05. Die Kettenlängenverteilung sowie die charakteristischen Kettenlängenverhältnisse wurden mittels GC bestimmt.

In Abhängigkeit vom Umsetzungsgrad der Ester enthalten die erfindungsgemäß eingesetzten Reiskleiewachsoxidate genuine Ester ($C_{46}$-$C_{62}$) in Mengen kleiner 50 Gew.-%, bevorzugt kleiner 25 Gew.-%, besonders bevorzugt kleiner 15 Gew.-%. Ferner enthalten die erfindungsgemäß eingesetzten Reiskleiewachsoxidate kleine Mengen an aliphatische Disäuren ($C_{10}$ bis $C_{32}$), in Abhängigkeit vom Umsetzungsgrad von 5 bis 15 Gew.-%. Daneben können die erfindungsgemäß eingesetzten Reiskleiewachsoxidate auch kleine Mengen aliphatischer Alkanole ($C_{24}$ bis $C_{36}$) enthalten.

Weitere nicht näher spezifizierte Bestandteile (z. B. Phosphorlipide, Sterolderivate, Sterolester, Oryzanole, Tocotrienole, Glykolipide, Squalene etc.) aus dem rohen oder raffinierten Reiskleiewachsrohstoff sowie die daraus entstandenen Oxidate können in Mindermengenkonzentrationen bis zu 12 Gew.-% auftreten.
In Abhängigkeit des Ölgehalts (Reiskleieöl), welches prozessbedingt im rohen oder raffinierten Reiswachsrohstoff vorhanden ist, erhöht sich der Anteil an kurzen freien Fettsäuren ($C_{16}$ bis $C_{20}$). Bevorzugt umfassen die erfindungsgemäß eingesetzten Reiskleiewachsoxidate daher auch den ölstämmigen Anteil an freien Fettsäuren ($C_{14}$ bis $C_{20}$) bis zu maximal 50 Gew.-%, bevorzugt bis zu maximal 30 Gew.-%, besonders bevorzugt bis zu maximal 5 Gew.-%.

Erfindungsgemäß bestehen die Reiskleiewachsoxidate mindestens zu 85 Gew.-% aus

a) 40 bis 90 Gew.-% freie aliphatische Carbonsäuren $C_{16}$-$C_{36}$ (wachsstämmig),
b) 1 bis 50 Gew.-% freie aliphatische Alkohole $C_{24}$-$C_{36}$ (wachsstämmig),
c) 3 bis 15 Gew.-% freie aliphatische Disäuren $C_{10}$-$C_{30}$ (wachsstämmig),
d) 0 bis 50 Gew.-% genuinen Estern $C_{44}$-$C_{62}$ (wachsstämmig),
e) 0 bis 50 Gew.-% freie aliphatische Carbonsäuren $C_{14}$-$C_{20}$ (öl- und/oder fettstämmig),
f) 0 bis 12 Gew.-% weitere im Reiswachs vorhandene natürliche Bestandteile.

Erfindungsgemäß bevorzugt bestehen die Reiskleiewachsoxidate mindestens zu 85 Gew.-% aus

a) 60 bis 90 Gew.-% freie aliphatische Carbonsäuren $C_{16}$-$C_{36}$ (wachsstämmig),
b) 1 bis 20 Gew.-% freie aliphatische Alkohole $C_{24}$-$C_{36}$ (wachsstämmig),
c) 5 bis 15 Gew.-% freie aliphatische Disäuren $C_{10}$-$C_{30}$ (wachsstämmig),
d) 0 bis 30 Gew.-% genuinen Estern $C_{44}$-$C_{62}$ (wachsstämmig),
e) 0 bis 30 Gew.-% freie aliphatische Carbonsäuren $C_{14}$-$C_{20}$, (öl- und/oder fettstämmig),
f) 0 bis 12 Gew.-% weitere im Reiswachs vorhandene natürliche Bestandteile.

Erfindungsgemäß besonders bevorzugt bestehen die Reiskleiewachsoxidate mindestens zu 85 Gew.-% aus

a) 70 bis 90 Gew.-% freie aliphatische Carbonsäuren $C_{16}$-$C_{36}$ (wachsstämmig),
b) 1 bis 5 Gew.-% freie aliphatische Alkohole $C_{24}$-$C_{36}$ (wachsstämmig),
c) 5 bis 12 Gew.-% freie aliphatische Disäuren $C_{10}$-$C_{30}$ (wachsstämmig),
d) 0 bis 20 Gew.-% genuinen Estern $C_{44}$-$C_{62}$ (wachsstämmig),
e) 0 bis 30 Gew.-% freie aliphatische Carbonsäuren $C_{14}$-$C_{20}$, (öl- und/oder fettstämmig),
f) 0 bis 12 Gew.-% weitere im Reiswachs vorhandene natürliche Bestandteile.

Bevorzugt zeichnen sich die erfindungsgemäßen Reiskleiewachsoxidate durch einen Tropfpunkt gemessen nach DIN ISO 2176 zwischen 70 °C und 90 °C, bevorzugt zwischen 75 °C und 85 °C aus.
Bevorzugt zeichnen sich die erfindungsgemäß eingesetzten Reiskleiewachsoxidate durch eine Esterzahl von maximal 130, bevorzugt maximal 60, besonders bevorzugt maximal 40 aus. Die Esterzahl berechnet sich in bekannter Weise aus der Differenz von Verseifungszahl und Säurezahl.

Bevorzugt zeichnen sich die erfindungsgemäß eingesetzten Reiskleiewachsoxidate durch eine besonders helle bis weiße Farbe aus. Die Farbe lässt sich anhand der lodfarbzahl gemessen nach DIN 6162 quantifizieren und liegt bei den erfindungsgemäß eingesetzten Reiskleiewachsoxidaten bei kleiner 20, bevorzugt kleiner 10, besonders bevorzugt kleiner 5.

Vorzugsweise erhöht sich durch die Oxidation des Reiskleiewachsrohstoffs die Verseifungszahl gemessen nach DIN ISO 3681 um mindestens 50 %, bevorzugt um mindestens 80 %, besonders bevorzugt um mindestens 150 %. Die Erhöhung der Verseifungszahl kann mechanistisch durch die Spaltung der Wachsester und der anschließenden Oxidation der Wachsalkohole zu Säuren erklärt werden. Zusätzlich dazu wird ein Teil ungesättigter Kohlenstoff-Kohlenstoffbindungen durch die Chromsäure gespalten und ebenfalls zu Säuren aufoxidiert. Damit ist die Verseifungszahl auch eine Maßzahl für die tatsächlich stattgefundene Oxidation in Abgrenzung zur Verseifung, bei der sich die Verseifungszahl bekanntlich nicht ändert, und in Abgrenzung zu anderen Bleichverfahren bei denen es lediglich zu einer Aufhellung des Produkts kommt. Beispielsweise bewirkt die Bleichung von Reiskleiewachs mit Wasserstoffperoxid keine chemische Modifikation des Wachses im Sinne der Erfindung, da hier lediglich verfärbende Verunreinigungen und Nebenbestandteile beseitigt werden, ohne dass die eigentliche Wachsstruktur verändert wird. Aufgrund der schlechten Emulgierbarkeit und der damit zu geringen Grenzfläche (Anmerkung: Bei der Oxidation von Naturwachsen mit Chromschwefelsäure handelt es sich um eine Grenzflächenreaktion) von Chromschwefelsäure mit dem Reiswachs findet keine signifikante Erhöhung der Verseifungszahl sowie der Säurezahl statt.

Die erfindungsgemäß eingesetzten Reiskleiewachsoxidate können zweistufig durch Verseifung unter Druck, optional anschließender Neutralisation und darauffolgender Oxidation mit Chromschwefelsäure hergestellt werden. Die Verseifung unter Druck verläuft bei 1 bis 20 bar, bevorzugt bei 5 bis 15 bar, besonders bevorzugt bei 10 bis 15 bar und erhöhter Temperatur bei 80 bis 250 °C , bevorzugt bei 180 °C bis 225 °C mit halbmolar bis 1,5-fachen Überschuss an NaOH. Die drastischen Reaktionsbedingungen sind ebenfalls der schlechten Emulgierbarkeit von Reiskleiewachs geschuldet. Eine drucklose Verseifung gelingt nur unter deutlichem Überschuss von KOH oder NaOH und, wie bei der Bestimmung der Verseifungszahl, zusätzlichem Lösungsmitteleinsatz (Xylol).

Ersteres verursacht bei der anschließenden Neutralisation eine deutlich erhöhte Salzfracht. Die anschließende Oxidation kann kontinuierlich mittels des Gersthofener Verfahrens als auch im Batchverfahren mit Kaliumdichromat in Schwefelsäure durchgeführt werden. Entsprechende Herstellprozesse sind etwa in Ullmann's Encyclopedia of Industrial Chemistry, 2000, Waxes sowie in

DE 10231886 sowie in Vladimir Vcelák, Chemie und Technologie des Montanwachses, 1959, Teil B: Veredelung des Montanwachses, S. 458 ff beschrieben.

Die erfindungsgemäß eingesetzten Reiskleiewachsoxidate können einstufig durch Oxidation mit Chromschwefelsäure ohne vorherige Verseifung unter gleichzeitiger Zugabe eines Emulsionsvermittlers hergestellt werden. Der Emulsionsvermittler wird zu maximal 10 Gew.-%, bezogen auf das eingesetzte Ausgangswachs, bevorzugt zu maximal 5 Gew.-%, besonders bevorzugt zu maximal 1 Gew.-% eingesetzt. Als Emulsionsvermittler werden beispielsweise säurestabile und, zur besseren Abtrennbarkeit, wasserlösliche Emulgatoren (wie z. B. Aklansulfonate, perfluorierte Aklansulfonsäuren, Nonafluoro-1-butansulfonsäure, etc.), Tenside, polymere Tenside, stickstoffhaltige Kationentenside, Phasentransferkatalysatoren sowie auch Metallsalze (wie z. B. $AlCl_3$) oder Salzsäure eingesetzt. Weitere zur Herstellung von Reiskleiewachsoxidaten geeignete Emulsionsvermittler können etwa in Ullmann's Encyclopedia of Industrial Chemistry, 2000, Emulsions gefunden werden. Unterstützend kann die Grenzfläche der Reaktion bei dieser Ausführungsform durch starkes mechanisches Dispergieren erhöht werden. Im Grenzfall einer ausreichend hohen mechanischen Dispergierwirkung, kann auf einen Emulsionsvermittler verzichtet werden. Besonders geeignet zur Herstellung der erfindungsgemäßen Reiskleiewachsoxidate ist die unter starker mechanischer Dispergierung durchgeführte Oxidation mit Chromschwefelsäure.

Mit den beiden genannten Herstellverfahren können nicht nur Reiskleiewachs auf Säurezahlen von mindestens 70 mg KOH/g, bevorzugt mindestens 100 mg KOH/g, besonders bevorzugt mindestens 140 mg KOH/g oxidiert werden, sondern auch andere schlecht oxidierbare Naturwachse, wie z. B. Sonnenblumenwachs.

Die erfindungsgemäßen Reiskleiewachsoxidate können nach erfolgter Chromsäureoxidation unabhängig von der Herstellungsvariante gegebenenfalls durch Waschen oder Zentrifugieren von Chromseifen befreit werden. Bevorzugt genügt die Reinheit der erfindungsgemäßen Reiskleiewachsoxidate dem Deutschen Arzneibuch für Cera montanglykoli.

Als Reiswachsausgangsstoff zur Herstellung von Reiskleiewachsoxidaten können aus Reiskleie durch beliebige Trennverfahren gewonnene wachsartige Bestandteile eingesetzt werden. Bevorzugt sind dabei die aus Reiskleieöl in bekannter Weise, z. B. durch Ausfrieren oder Extrahieren, isolierten Wachsanteile. Diese können als solche und/oder nach mechanischer bzw. physikalischer Reinigung und/oder nach Bleichung mittels Wasserstoffperoxid verwendet werden. Bevorzugt werden die erfindungsgemäßen Reiskleiewachsoxidate durch Chromsäureoxidation unabhängig von den oben beschriebenen Herstellungsvarianten wahlweise aus rohem, nicht raffiniertem oder raffiniertem Reiskleiewachs hergestellt. Insbesondere enthält das Reiskleiewachs Spuren bis deutliche Mengen an Reiskleieöl von maximal 50 Gew.-%, bevorzugt maximal 30 Gew.-%, besonders bevorzugt maximal 5 Gew.-%.

Geeignete Carnaubawachsoxidate und deren Herstellung sind beispielsweise in DE 102 31 886 (B4) beschrieben. Weitere Naturwachse wie z. B. Candelillawachs, Zuckerrohrwachs, Carnaubawachs, etc. als Rohstoffbasis für erfindungsgemäß verwendete Naturwachsoxidate sind etwa in Ullmann's Encyclopedia of Industrial Chemistry, 5th Ed. 1996, Vol. A28, Seiten 110-122 beschrieben.

Für ein typisches Candellilawachs (vor der Oxidation) liegt nach Ullmann (Seite 122, Tabelle 4) der Anteil der Säuren bei 0,3 ($C_{22}$), 0,1 ($C_{24}$), 0,3 ($C_{26}$), 3,2 ($C_{28}$), 36,4 ($C_{30}$), 48,6 ($C_{32}$) und 8,0 ($C_{34}$), der Anteil der Alkohole 2,4 ($C_{26}$), 17,4 ($C_{28}$), 58,0 ($C_{30}$) und 19,0 ($C_{32}$) (Gew.-%, jeweils für die Gesamtmenge der Säuren oder Alkohole).

Amidamine der Formel (I) sind durch Umsetzung eines Naturwachsoxidats erhältlich (d.h. die Reaktionsmischung enthält keine (0 Gew.-%) weiteren ($C_{18}$-$C_{22}$)-aliphatischen Carbonsäuren.

Die Erfindung umfasst auch eine kosmetische Zubereitung, enthaltend die oben beschriebene Kombinationen. Dabei ist es bevorzugt, wenn solche Zubereitungen zusätzlich wenigstens 0,01 Gew.-% Natriumchlorid enthalten. Besonders bevorzugt ist es, wenn in erfindungsgemäßen Zubereitungen wenigstens 0,3 Gew.-% an kationischen Tensiden enthalten sind, bezogen auf das Gesamtgewicht der Zubereitung. Bevorzugte kationische Tenside sind quartäre Ammonium-Salze, wie Di-($C_8$-$C_{22}$)-Alkyl-dimethylammoniumchlorid oder -bromid, vorzugsweise Di-($C_8$-$C_{22}$)-Alkyl-dimethylammoniumchlorid oder -bromid; ($C_8$-$C_{22}$)-Alkyldimethylethylammoniumchlorid oder -bromid; ($C_8$-$C_{22}$)-Alkyltrimethylammoniumchlorid oder -bromid, vorzugsweise Cetyltrimethylammoniumchlorid oder -bromid und ($C_8$-$C_{22}$)-Alkyltrimethylammoniumchlorid oder -bromid; ($C_{10}$-$C_{24}$)-Alkyldimethylbenzylammoniumchlorid oder -bromid, vorzugsweise ($C_{12}$-$C_{18}$)-Alkyldimethylbenzylammoniumchlorid, ($C_8$-$C_{22}$)-Alkyldimethylhydroxyethylammoniumchlorid,- phosphat, -sulfat, -lactat, ($C_8$-$C_{22}$)-Alkylamidopropyltrimethylammoniumchlorid, -methosulfat, N,N-bis(2-$C_8$-$C_{22}$-Alkanoyloxyethyl)-dimethylammoniumchlorid, -methosulfat, N,N-bis(2-$C_8$-$C_{22}$-Alkanoyloxyethyl)hydroxyethylmethylammoniumchlorid, -methosulfat.

Die Menge der kationischen Tenside beträgt bevorzugt von 0,1 bis 10,0 Gew.-%, besonders bevorzugt von 0,5 bis 7,0 Gew.-% , insbesondere besonders bevorzugt von 1,0 bis 5,0 Gew.-%, ganz besonders bevorzugt von 1 bis 3 Gew.-%, bezogen auf die fertigen Zubereitungen.

Weiterhin ist es besonders bevorzugt, wenn in erfindungsgemäßen Zubereitungen wenigstens 0,1 Gew.-% an kationischen Polymeren enthalten sind, bezogen auf das Gesamtgewicht der Zubereitung. Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-6, Polyquaternium-80, Polyquaternium-22, Polyquaternium-39, Polyquaternium-28, Polyquaternium-2, Polyquaternium-10, Polyquaternium-11, sowie Polyquaternium 37 & mineral oil & PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen Zubereitungen können ein oder mehrere der oben genannten kationischen Polymere in Mengen von 0,1 bis 5,0 Gew.-%, vorzugsweise von 0,2 bis 3,0 Gew.-%, besonders bevorzugt von 0,5 bis 2,3 Gew.-%, und ganz besonders bevorzugt von 1 bis 2,0 Gew.-%, bezogen auf die fertigen Zubereitungen, enthalten.

Die Erfindung umfasst auch die Verwendung erfindungsgemäßer Zubereitungen als Haarpflegemittel, als Haarspülung oder als Haarshampoo. Haarpflegemittel im Sinne dieser Schrift umfassen Zubereitungen zur Behandlung oder Pflege von Haaren, beispielsweise um Shampoos, rinse-off-Haarconditionierer, Cremespülungen, Klarspülungen, Haarkuren, Haarfärbe- und Haartönungsmittel, Dauerwellmittel, Haargele, Haarconditionierer in Aerosol-, Spray- und Fluidform, sowie um Shampoos mit zusätzlichem Pflegeeffekt (2 in 1 Shampoos).

Weiter bevorzugt sind erfindungsgemäße Zubereitungen, enthaltend einen oder mehrere aliphatische Alkohole der Formel (III),

$$R^3\text{-OH} \qquad \text{(III)}$$

worin $R^3$ eine aliphatische gesättigte oder ungesättigte $C_8$-$C_{30}$-Kohlenwasserstoffgruppe ist und worin lineare aliphatische Kohlenwasserstoffgruppen 80 Gew.-% oder mehr ausmachen; dabei ist es besonders bevorzugt, wenn erfindungsgemäße Zubereitungen Cetyl- und/oder Stearylalkohol enthalten. Besonders bevorzugt ist es weiter, wenn erfindungsgemäße Zubereitungen eine oder mehrere organische Säuren enthalten, ganz besonders bevorzugt Citronensäure, Milchsäure und/oder Glutaminsäure. Dadurch werden die Verdickungseigenschaften (Gelbildungsfähigkeit) und Stabilität der erfindungsgemäßen Zubereitungen weiter verbessert.

Die organische Säure ist bevorzugt eine organische $C_{10}$ oder kürzere Säure und Beispiele umfassen Säuren mit $C_{10}$ oder kürzeren Alkylgruppen wie Alkylphosphorsäuren, Alkylsulfonsäuren, Alkylschwefelsäuren etc.; saure Aminosäuren wie L-Glutaminsäure und L-Asparaginsäure; Glutaminsäure; aromatische Säuren wie Benzoesäure und p-Toluolsulfonsäure; Hydroxysäuren und Dicarbonsäuren. Die Hydroxysäuren umfassen Hydroxymonocarbonsäuren wie

Glykolsäure, Milchsäure, Glycerinsäure, Gluconsäure und Pantothensäure; Hydroxydicarbonsäuren wie Apfelsäure und Weinsäure und Hydroxytricarbonsäuren wie Zitronensäure. Die Dicarbonsäuren umfassen zum Beispiel Oxalsäure, Malonsäure, Maleinsäure, Succinsäure und Glutarsäure. Hydroxysäuren und Glutaminsäure sind besonders bevorzugt, wobei Citronensäure Glykolsäure, Milchsäure, Apfelsäure und Glutaminsäure besonders bevorzugt sind.

Wenn die organische Säure erfindungsgemäßen Zubereitungen zugesetzt wird, können die Komponenten (a) und die organische Säure getrennt oder nach vorhergehender Bildung eines Säuresalzes des Amidamins (I) vermischt werden. Die Menge der organischen Säure ist bevorzugt 0,3 bis 10 Mol, mehr bevorzugt 0,5 bis 5 Mol pro Mol der Komponente (a).

Erfindungsgemäße Zubereitungen dieser Erfindung werden nach teilweiser oder vollständiger Umwandlung in ein Salz durch Regulieren des pH-Wertes und bevorzugt bei einem pH-Wert von 2 bis 8, mehr bevorzugt 3 bis 6 verwendet.

Die konditionierende Wirkung der erfindungsgemäßen Haarpflegemittel kann durch Zugabe von N-modifizierten Silikonen weiter gesteigert werden.

In einer weiteren bevorzugten Ausführungsform der Erfindung enthalten die erfindungsgemäßen Zubereitungen Alkylmethicone, Alkyldimethicone oder eine oder mehrere Amodimethicone. Amodimethicone sind Siloxane Polymere, die mit aminofunktionellen Gruppen gepfropft sind. Amodimethicone sind z. B. unter dem Handelsnamen Dow Corning 2-8566 Amino Fluid (Dow Corning Corporation), Mirasil ADME (Rhodia), SilCare® Silicone SEA (Clariant) oder Wacker-Belsil ADM 1100 (Wacker Chemie AG) bekannt, haben ein Molekulargewicht zwischen 800 und 260000 g/mol und entsprechen allgemein der Formel (IV).

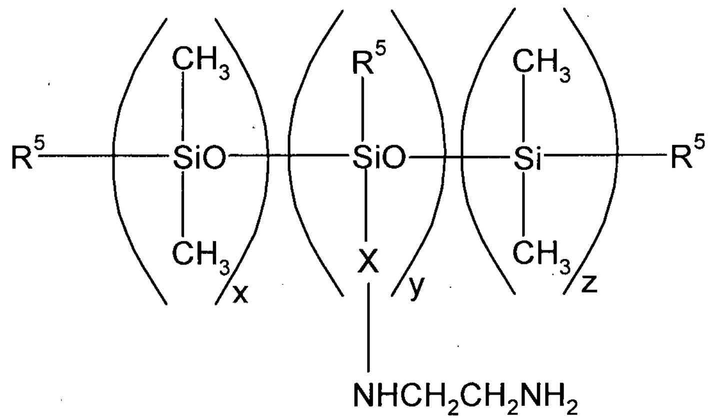

(IV)

worin

| | |
|---|---|
| $R^5$ | -OH oder -$CH_3$, |
| X | eine lineare oder verzweigte $C_1$-$C_6$-Alkylengruppe und |
| x, y und z | jeweils unabhängig voneinander eine Zahl von 1 bis 5500, bevorzugt von 50 bis 500, sind. |

In einer besonders bevorzugten Ausführungsform der Erfindung enthalten die erfindungsgemäßen Zubereitungen eine oder mehrere der oben genannten Verbindungen der Formel (IV) und vorzugsweise, bezogen auf die gesamte Zubereitung, 0,1 bis 5,0 Gew.-% an einer oder mehrerer Verbindungen der Formel (IV).

Die Kompatibilität mit weiteren Inhaltsstoffen sowie das Hautgefühl und der Antistatik-Effekt der Zubereitung kann durch Zugabe von einer oder mehreren Verbindungen der Formel (V)

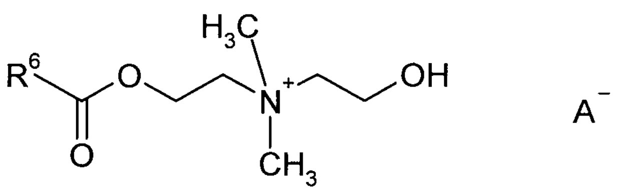

(V)

verbessert werden, worin

$R^6CO$ eine lineare oder verzweigte, vorzugsweise eine lineare, des Weiteren bevorzugt eine gesättigte, Acylgruppe mit 18 bis 24 C-Atomen, vorzugsweise mit 18 bis 22 C-Atomen, bedeutet und

$A^-$ ein Gegenion ist.

In einer weiteren bevorzugten Ausführungsform der Erfindung enthalten die erfindungsgemäßen Zubereitungen eine oder mehrere Verbindungen der Formel (V)

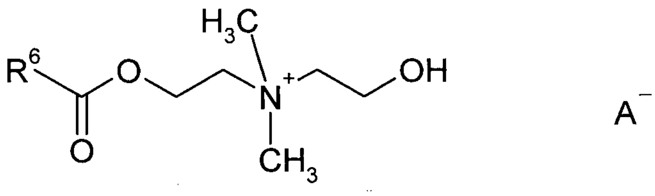

(V)

worin

$R^6CO$ eine lineare oder verzweigte, vorzugsweise eine lineare, des Weiteren bevorzugt eine gesättigte, Acylgruppe, mit 18 bis 24 C-Atomen, vorzugsweise mit 18 bis 22 C-Atomen, bedeutet und

$A^-$ ein Gegenion ist

und vorzugsweise, bezogen auf die gesamte Zubereitung, von 0,1 bis 5,0 Gew.-% an einer oder mehreren Verbindungen der Formel (V).

Das Gegenion $A^-$ der Formel (V) ist vorzugsweise ausgewählt aus Chlorid, Bromid, Methosulfat $MeSO_4^-$, Tosylat, Phosphat, Sulfat, Hydrogensulfat, Lactat und Citrat und besonders bevorzugt ausgewählt aus Chlorid und Methosulfat $MeSO_4^-$.

Bei den erfindungsgemäßen Zubereitungen handelt es sich bevorzugt um Emulsionen oder Dispersionen, besonders bevorzugt um Dispersionen.

In einer weiteren bevorzugten Ausführungsform der Erfindung enthalten die erfindungsgemäßen Zubereitungen einen oder mehrere nichtionische Emulgatoren, und vorzugsweise, bezogen auf die gesamte Zubereitung, von 0,1 bis 5,0 Gew.-% an einem oder mehreren nichtionischen Emulgatoren.

Als nichtionogene Emulgatoren kommen vorzugsweise in Betracht: Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; ($C_{12}$-$C_{18}$)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxid-Anlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole, Cetylstearylalkohole, insbesondere Polyethylenglykol(13)stearylether, Polyethylenglykol(14)stearylether, Polyethylenglykol(15)stearylether, Polyethylenglykol(16)stearylether, Polyethylenglykol(17)stearylether, Polyethylenglykol(18)stearylether, Polyethylenglykol(19)stearylether, Polyethylenglykol(20)stearylether, Polyethylenglykol(12)isostearylether, Polyethylenglykol(13)isostearylether, Polyethylenglykol(14)isostearylether, Polyethylenglykol(15)isostearylether, Polyethylenglykol(16)isostearylether, Polyethylenglykol(17)isostearylether, Polyethylenglykol(18)isostearylether, Polyethylenglykol(19)isostearylether, Polyethylenglykol(20)isostearylether, Polyethylenglykol(13)cetylether, Polyethylenglykol(14)cetylether, Polyethylenglykol(15)cetylether, Polyethylenglykol(16)cetylether, Polyethylenglykol(17)cetylether, Polyethylenglykol(18)cetylether, Polyethylenglykol(19)cetylether, Polyethylenglykol(20)cetylether, Polyethylenglykol(13)isocetylether, Polyethylenglykol(14)isocetylether, Polyethylenglykol(15)isocetylether, Polyethylenglykol(16)isocetylether, Polyethylenglykol(17)isocetylether, Polyethylenglykol(18)isocetylether, Polyethylenglykol(19)isocetylether, Polyethylenglykol(20)isocetylether, Polyethylenglykol(12)oleylether, Polyethylenglykol(13)oleylether, Polyethylenglykol(14)oleylether, Polyethylenglykol(15)oleylether, Polyethylenglykol(12)laurylether, Polyethylenglykol(12)isolaurylether, Polyethylenglykol(13)cetylstearylether, Polyethylenglykol(14)cetylstearylether, Polyethylenglykol(15)cetylstearylether, Polyethylenglykol(16)cetylstearylether, Polyethylenglykol(17)cetylstearylether, Polyethylenglykol(18)cetylstearylether, Polyethylenglykol(19)cetylstearylether.

Ebenso bevorzugt sind Fettsäureethoxylate, gewählt aus der Gruppe der ethoxylierten Stearate, Isostearate und Oleate, insbesondere Polyethylenglykol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat,

Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders Polyethylenglykol(20)sorbitanmonolaurat, Polyethylenglykol(20)sorbitanmonostearat, Polyethylenglykol(20)sorbitanmonoisostearat, Polyethylenglykol(20)sorbitanmonopalmitat, Polyethylenglykol(20)sorbitanmonooleat.

Weiter in Betracht kommen Glycerylmonostearat, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glykoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglykol(2)stearylether (Steareth-2), Alkylmethiconcopolyole und Alkyl-Dimethiconcopolyole, insbesondere Cetyldimethiconcopolyol, Laurylmethiconcopolyol.

Die kosmetischen Zubereitungen können als weitere Hilfs- und Zusatzstoffe alle üblichen Tenside, Ölkörper, kationischen Polymere, Filmbildner, Verdickungs- und Gelierungsmittel, Überfettungsmittel, antimikrobielle und biogene Wirkstoffe, feuchtigkeitsspendende Mittel, Stabilisatoren, Konservierungsmittel, Perlglanzmittel, Farb- und Duftstoffe, enthalten.

Als Tenside können kationische, nichtionische, amphotere und/oder zwitterionische Tenside verwendet werden.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics®); Fettsäurealkanolamide, (Fettsäureamidpolyethylenglykole); Saccharoseester; Sorbitester und Sorbitanester und deren Polyglykolether, sowie $C_8$-$C_{22}$-Alkylpolyglucoside.

Die Menge der nichtionischen Tenside in den erfindungsgemäßen Zubereitungen (z. B. im Falle von Rinse-off-Produkten) liegt bevorzugt im Bereich von 1,0 bis 20,0 Gew.-%, besonders bevorzugt von 2,0 bis 10,0 % und insbesondere bevorzugt von 3,0 bis’7,0 Gew.-%.

Weiterhin können die erfindungsgemäßen Zubereitungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat- Gruppe. Bevorzugte Amphotenside sind N-($C_{12}$-$C_{18}$)-Alkyl-β-aminopropionate und N-($C_{12}$-$C_{18}$)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die $C_{10}$- bis $C_{18}$-Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die $C_{12}$- bis $C_{18}$- Dimethylaminohexanoate bzw. die $C_{10}$- bis $C_{18}$- Acylamidopropandimetylbetaine.

Die Menge der amphoteren Tenside und/oder Betaintenside beträgt bevorzugt von 0,5 bis 20,0 Gew.-% und besonders bevorzugt von 1,0 bis 10,0 Gew.-%.

Bevorzugte Tenside sind, Cocoamidopropylbetain, Alkylbetaine wie Coco-Betain, Natriumcocoylglutamat und Lauroamphoacetat.

In einer bevorzugten Ausführungsform enthalten die erfindungsgemäßen Zubereitungen zusätzlich noch als schaumverstärkende Mittel Co-Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die Ölkörper können vorteilhafterweise ausgewählt werden aus den Gruppen der Triglyceride, natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren oder

aus der Gruppe der Alkylbenzoate, sowie natürliche oder synthetische Kohlenwasserstofföle.

In Betracht kommen Triglyceride von linearen oder verzweigten, gesättigten oder ungesättigten, gegebenenfalls hydroxylierten, $C_6$-$C_{36}$-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol® 318. Auch gehärtete Triglyceride sind erfindungsgemäß bevorzugt. Auch Öle tierischen Ursprungs, beispielweise Rindertalg, Perhydrosqualen, Lanolin können eingesetzt werden.

Eine weitere Klasse von erfindungsgemäß bevorzugten Ölkörpern sind die Benzoesäureester von linearen oder verzweigten $C_{8-22}$-Alkanolen, z. B. die Handelsprodukte Finsolv® SB (Isostearylbenzoat), Finsolv® TN ($C_{12}$-$C_{15}$-Alkylbenzoat) und Finsolv® EB (Ethylhexylbenzoat).

Eine weitere Klasse von erfindungsgemäß bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol® OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Butyl-n-octylether, iso-Pentyl-n-octylether und 2-Methyl-pentyl-n-octylether sowie Di-tert.-butylether und Diisopentylether.

Ebenso in Betracht kommen verzweigte gesättigte oder ungesättigte Fettalkohole mit 6 bis 30 Kohlenstoffatomen, z. B. Isostearylalkohol, sowie Guerbetalkohole.

Eine weitere Klasse von erfindungsgemäß bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der $\beta$-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von $C_{12}$-$C_{15}$-Fettalkoholen besonders bevorzugt. Ester dieses Typs sind im Handel erhältlich, z. B. unter dem Handelsnamen Cosmacol® der EniChem, Augusta Industriale.

Eine weitere Klasse von erfindungsgemäß bevorzugten Ölkörpern sind Dicarbonsäureester von linearen oder verzweigten $C_2$-$C_{10}$-Alkanolen, wie Di-n-butyladipat (Cetiol® B), Di-(2-ethylhexyl)-adipat und Di-(2-ethylhexyl)-succinat sowie Diolester wie Ethylenglykol-dioleat, Ethylenglykoldiisotridecanoat, Propylenglykol-di-(2-ethylhexanoat), Propylenglykoldiisostearat, Propylenglykol-dipelargonat, Butandioldiisostearat und Neopentylglykoldicaprylat sowie Diisotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol® CC).

Eine weitere Klasse von erfindungsgemäß bevorzugten Ölkörpern sind die Ester von Dimeren ungesättigter $C_{12}$-$C_{22}$-Fettsäuren (Dimerfettsäuren) mit einwertigen linearen, verzweigten oder cyclischen $C_2$-$C_{18}$-Alkanolen oder mit mehrwertig linearen oder verzweigten $C_2$-$C_6$-Alkanolen.

Eine weitere Klasse von erfindungsgemäß bevorzugten Ölkörpern sind Kohlenwasserstofföle, zum Beispiel solche mit linearen oder verzweigten, gesättigten oder ungesättigten $C_7$-$C_{40}$-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl®-Serie, Squalan, Squalen, und alicyclische Kohlenwasserstoffe, z. B. das Handelsprodukt 1,3-Di-(2-ethyl-hexyl)-cyclohexan (Cetiol® S), Ozokerit, und Ceresin.

An Silikonölen bzw. -wachsen stehen vorzugsweise zur Verfügung Dimethylpolysiloxane und Cyclomethicone, Polydialkylsiloxane $R_3SiO(R_2SiO)_xSiR_3$, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare® Silicone 41M65, SilCare® Silicone 41M70, SilCare® Silicone 41M80 (Clariant) erhältlichen Dimethicone, Stearyldimethylpolysiloxan, $C_{26}$-$C_{24}$-Alkyldimethylpolysiloxan, $C_{24}$-$C_{28}$-Alkyldimethylpolysiloxan, aber auch die unter SilCare® Silicone 41M40, SilCare® Silicone 41M50 (Clariant) erhältlichen Methicone, weiterhin Trimethylsiloxysilicate $[(CH_2)_3SiO)_{1/2}]_x[SiO_2]_y$, wobei x für eine Zahl von 1 bis 500 und y für eine Zahl von 1 bis 500 steht, Dimethiconole $R_3SiO[R_2SiO]_xSiR_2OH$ und $HOR_2SiO[R_2SiO]_xSiR_2OH$, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Des Weiteren können die erfindungsgemäßen Zubereitungen Filmbildner enthalten, die je nach Anwendungszweck ausgewählt sind aus Salzen der Phenylbenzimidazolsulfonsäure, wasserlöslichen Polyurethanen, beispielsweise $C_{10}$-Polycarbamylpolyglycerylester, Polyvinylalkohol, Polyvinylpyrrolidoncopolymeren wie PVP/Hexandecene oder PVP/Eicosene Copolymer, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure und

Polyethylenglykolethern von Fettalkoholen, wie Acrylat/Steareth-20-Methacrylat-Copolymer, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex® A 60 (Clariant) erhältlich, sowie polymeren Aminoxiden, beispielsweise unter den Handelsnamen Diaformer Z-711, 712, 731, 751 erhältliche Vertreter.

Die erfindungsgemäßen Zubereitungen können einen oder mehrere Filmbildner in Mengen von 0,1 bis 10,0 Gew.-%, vorzugsweise von 0,2 bis 5,0 Gew.-% und besonders bevorzugt von 0,5 bis 3,0 Gew.-%, bezogen auf die fertigen Zubereitungen, enthalten.

Die gewünschte Viskosität der Zubereitungen kann durch Zugabe von Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Desweiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

Bevorzugt enthalten die erfindungsgemäßen Zubereitungen von 0,01 bis 20,0 Gew.-%, besonders bevorzugt von 0,1 bis 10,0 Gew.-%, insbesondere bevorzugt von 0,2 bis 3,0 Gew.-% und ganz besonders bevorzugt von 0,4 bis 2,0 Gew.-% an Verdickern bzw. Geliermitteln.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zubereitungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zubereitungen.

Die erfindungsgemäßen Zubereitungen können des Weiteren biogene Wirkstoffe ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol®, Allantoin®, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten; sowie weitere Wirkstoffe wie zum Beispiel Q10, Hyaluron, Keratin, Proteinhydrolysate, Pflanzenextrakte, Öle wie z. B. Arganöl, Avocadoöl, Babassuöl, Mandelkernöl, Aprikosenöl.

Die erfindungsgemäßen kosmetischen Zubereitungen können auch die für solche Anwendungen üblichen UV Filter und/oder Antioxidantien enthalten.

Die erfindungsgemäßen Zubereitungen können biogene Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zubereitungen, enthalten.

Die erfindungsgemäßen Zubereitungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.-% und insbesondere bevorzugt in Mengen von 0,1 bis 10,0 Gew.-% enthalten. Als deodorierende Stoffe bevorzugt sind Allantoin und Bisabolol. Diese werden vorzugsweise in Mengen von 0,0001 bis 10,0 Gew.-% eingesetzt.

Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z. B. Magnesium-, Aluminium- und/oder Zinkstearat eingesetzt werden, bevorzugt in Mengen von 0,1 bis 10,0 Gew.-%, vorzugsweise von 0,5 bis 8,0 Gew.-% und besonders bevorzugt von 1,0 bis 5,0 Gew.-%, bezogen auf die fertigen Zubereitungen.

Als Konservierungsmittel eignen sich beispielsweise Phenoxyethanol, Formaldehydlösung, Parabene, Pentandiol und Sorbinsäure, sowie organische Säuren wie Benzoesäure, Salicylsäure, Anissäure, Methylisothiazolinone oder Velsan SC, Octopirox.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykoldistearate mit durchschnittlich 3 Glykoleinheiten.

Sofern die erfindungsgemäßen Zubereitungen perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zubereitungen enthalten.

Weiter können erfindungsgemäßen Zubereitungen Glimmer-Partikel (Mica) enthalten, etwa solche, die unter der Bezeichnung "Timiron" von der Gesellschaft Merck angeboten werde. Diese sind bevorzught in Anteilen von 0,5 - 2,5 Gew.-% in erfindungsgemäßen Zubereitungen enthalten.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z. B. die Ionone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die erfindungsgemäßen Zubereitungen sind vorzugsweise auf einen pH-Wert im Bereich von 2 bis 12, bevorzugt im Bereich von 3 bis 9, eingestellt.

Als Säuren zur pH-Wert Einstellung können neben den bevorzugten organischen Mineralsäuren, insbesondere HCl, als Laugen, anorganische Basen, insbesondere NaOH oder KOH verwendet werden.

Der Gesamtanteil an Hilfs- und Zusatzstoffen in den Mitteln bzw. in den erfindungsgemäßen Zubereitungen beträgt bevorzugt von 1,0 bis 50,0 Gew.-% und besonders bevorzugt von 5,0 bis 40,0 Gew.-%.

Die erfindungsgemäßen Zubereitungen sind in speziellen Ausführungsformen auch problemlos konfektionierbar.

Diese konfektionierbaren Massen können z. B. auch in vorteilhafter Weise als Grundlage für Cremespülungen verwendet werden.

Weitere Anwendungsgebiete sind zum Beispiel weitere Emulsionen wie Körperlotionen und Cremes.

Die nachfolgenden Beispiele dienen der Erläuterung der Erfindung, ohne sie jedoch darauf einzuschränken.

Die in den Beispielen angegebenen %-Angaben sind Gewichtsprozent (Gew.-%), sofern nicht explizit anders angegeben.

Beispiele:

A Ausgangsverbindungen

Im Folgenden wird beispielhaft die Herstellung von Reiskleiewachsoxidaten und Montanwachsoxidaten beschrieben. Das Verfahren kann ein- oder zweistufig erfolgen. Die einstufige Verfahrensweise bezieht sich hierbei auf den Verzicht auf eine vorhergehende Verseifung und nicht auf die Anzahl der Oxidationsstufen und damit angebotene Chromsäuremenge.

Substanzcharakterisierung:

Die im Folgenden aufgeführten Standardmethoden dienen zur Bestimmung der Kennzahlen von Reiskleiewachsen und Montanwachsen.

Tabelle 1: Zur Spezifikation von rohen Naturwachsen verwendete Standardmethoden

| | | Methode |
|---|---|---|
| Säurezahl | [mg KOH/g] | ISO 2114 |
| Verseifungszahl | [mg KOH/g] | ISO 3681 |
| DP | [°C] | ISO 2176 |
| DSC | [°C] | DIN EN ISO 11357-1 |
| DSC | [J/g] | DIN EN ISO 11357-1 |
| Ölgehalt | | AOCS Ja 4-46 |
| TGA | [wt.%,300°/+30min] | DIN EN ISO 11358 |
| Iodfarbzahl | | DIN 6162 |
| Iodzahl | [g $I_2$/100 g] | DGF M-IV 5 |

Die Kettenlängenverteilungen der Rohwachse wurden durch GF/UV-Trennungen ermittelt. Dazu wurden die Wachse zunächst unter definierten Bedingungen verseift, der UV-Anteil (Unverseifbares) extrahiert, der GF-Anteil (Gesamtfettsäuren) salzfrei gewaschen und beide Anteile separat per Gaschromatographie untersucht.

Als Vergleichssubstanzen dienten Wachssäuren und Wachsalkohole mit Kohlenstoffkettenlängen zwischen $C_6$ und $C_{36}$. Wachsester mit $C_{44}$ bis $C_{58}$ wurden durch Kombination der Modellsubstanzen dargestellt.

Um die Peaks der Wachs-GCs zu identifizieren wurde zu einer Reiskleiewachsprobe jeweils eine definierte Menge der einzelnen Komponenten zugegeben und eine deutliche Zunahme der Fläche des entsprechenden Peaks beobachtet.

Die Kettenlängenverteilungen der oxidierten Wachse wurden ebenfalls mittels Gaschromatographie bestimmt. Eine vorhergehende Verseifung ist in diesem Fall nicht notwendig, da die Ester bereits durch die Umsetzung mit Chromschwefelsäure in gespaltener Form vorliegen.

Tabelle 2 zeigt die Bedingungen unter denen die Gaschromatogramme der Wachse und Wachsderivate erstellt wurden.

Tabelle 2: GC Bedingungen

| | |
|---|---|
| Säule | Agilent Technologies HP-1 (DB-1)<br>Length 15 m I.D. 0,25 mm Film 0,10 µm |
| Detektor | 310 °C FID |
| Injektor | 300 °C Split 1:100 |
| Trägergas | Helium |
| Lösungsmittel | Toluol |
| Konzentration | 30 mg/ml |

(fortgesetzt)

| | |
|---|---|
| Säule | Agilent Technologies HP-1 (DB-1)<br>Length 15 m I.D. 0,25 mm Film 0,10 μm |
| Injektionsmenge | 1 μl |
| Temperaturprogramm | 40 bis 320 °C; 5 K/min; 50 min bei 320 °C halten |

Folgende Wachse dienten als Rohstoffe für die Wachsoxidate (Reiswachs Typ 1 - 4, Rohmontanwachs)

Tabelle 3: Verwendete Rohstoffe

| Rohstoff | Säurezahl [mgKOH/g] | Verseifungszahl [mgKOH/g] | Esterzahl | Tropfpunkt [°C] | Schmelzpunkt [°C] | Schmelzenthalpie [J/g] | Ölgehalt [%] | TGA [Gew.-% 300°C/ +30min] | Iodzahl [g $I_2$/ 100g] |
|---|---|---|---|---|---|---|---|---|---|
| Reiswachs Typ 1 | 6.3 | 81.4 | 75.1 | 78 | 80 | -206 | <2% | 4.2/14.6 | 4.8 |
| Reiswachs Typ 2 | 8.1 | 88.2 | 80.1 | 78 | 79 | -183 | 5% | 5.9/20.7 | 8.6 |
| Reiswachs Typ 3 | 1.3 | n.b. | n.b. | n.b. | 79 | -192 | <2% | 1.2/6.4 | |
| Reiswachs Typ 4 | 1.2 | 110 | 108.8 | 77 | n.b. | n.b. | >30% | 2.7/12.6 | 47 |
| Entharztes Rohmontanwachs | 28,0 | 80,9 | 52,9 | 86 | n.b. | n.b. | n.b. | n.b. | 30-45 |
| Carnaubawachs T4 (DE 10231886) | 11.1 | 83.4 | 72.3 | 84.9 | n.b. | n.b. | n.b. | n.b. | n.b. |
| n.b. = nicht bestimmt | | | | | | | | | |

Herstellungsbeispiel 1 Verseifung im Autoklaven

Ansatz:

| | |
|---|---|
| Reiskleiewachs | 500 g |
| NaOH | 35 g |
| Wasser | 300 g |

Durchführung:

In einem 1.5 l Druckgefäß mit Rührer und Temperaturfühler werden Wasser, NaOH und Reiskleiewachs vorgelegt. Der Autoklav wird verschlossen und das Reaktionsgemisch auf 220 °C erwärmt, es stellt sich ein Druck von 12 bar ein. Nach 6h lässt man den Druckreaktor im verschlossenen Zustand auf 80 °C abkühlen und entnimmt anschließend das Reaktionsgemisch.

Das Reaktionsgemisch wird bei 80 °C portionsweise mit Schwefelsäure (konz.) bis pH=3 versetzt und abgekühlt. Die wässrige Phase wird abgetrennt. Die organische Phase wird erneut geschmolzen und mit Wasser gewaschen bis das Waschwasser eine pH-neutrale Reaktion zeigt.

Tabelle 4: Verseifungen von Reiskleiewachs unter erhöhtem Druck bzw. bei Normaldruck.

| Versuchsnummer | | 1 | 2 | 3 | 4 |
|---|---|---|---|---|---|
| Reiswachs Typ 1 | [g] | 500 | | | |
| Reiswachs Typ 2 | [g] | | 500 | | |
| Reiswachs Typ 3 | [g] | | | 500 | |
| Reiswachs Typ 4 | [g] | | | | 500 |
| Natronlauge | [g] | 35.1 | 35.1 | 35.1 | 35.1 |
| Zusätze | | | | | |
| Wasser | [g] | 300 | 300 | 300 | 300 |
| Schwefelsäure, konz. | [g] | 49.7 | 49.7 | 49.7 | 49.7 |
| Bedingungen | | | | | |
| Reaktionsdauer | [h] | 6 | 6 | 6 | 6 |
| Temperatur | [°C] | 220 | 220 | 220 | 220 |
| Druck | [bar] | 12 | 12 | 12 | 12 |
| Prüfergebnisse | | | | | |
| Säurezahl | [mgKOH/g] | 76 | 72 | 74 | 78 |

Herstellungsbeispiel 2: Oxidation von verseiftem Reiskleiewachs

Ansatz:

| | |
|---|---|
| Verseiftes Reiskleiewachs | 500 g |
| Chromschwefelsäure (100 g $CrO_3$/L) | 2480 ml |

Durchführung:

In einem 5 l Reaktionsgefäß mit Rührer, Temperaturfühler, Tropftrichter und Rückflusskühler wird Chromschwefelsäure vorgelegt und auf 90 °C erwärmt. Anschließend wird geschmolzenes Reiskleiewachs portionsweise zugegeben. Das Reaktionsgemisch wird bei 110 °C für 10 h gerührt. Das Heizen sowie Rühren wird eingestellt. Sobald sich die Phasen getrennt haben wird die wässrige Phase abgetrennt.

Die organische Phase wird mit einer wässrigen Lösung von Oxalsäure und Schwefelsäure sowie Wasser chromfrei gewaschen, in warme Zentrifugengläser abgelassen und zentrifugiert.

Tabelle 5 zeigt die Produkte der Oxidation von verseiftem Reiskleiewachs (aus Tabelle 4). Die erreichte Säurezahl und damit der Umsatz der Ester ist höher als die Säurezahl die bei der einstufigen Oxidation von Rohmontanwachs erzielt wird.

Tabelle 5: Oxidation von verseiftem Reiskleiewachs und Rohmontanwachs

| Versuchsnummer | | 1 | 2 | 3 | 4 | 5 |
|---|---|---|---|---|---|---|
| Verseiftes Reiswachs Typ 1 | [g] | 500 | | | | |
| Verseiftes Reiswachs Typ 2 | [g] | | 500 | | | |
| Verseiftes Reiswachs Typ 3 | [g] | | | 500 | | |
| Verseiftes Reiswachs Typ 4 | [g] | | | | 500 | |
| Entharztes Rohmontanwachs | [g] | | | | | 155 |
| Chromschwefelsäure | [100 g $CrO_3$]/l] | 4960 | 4960 | 4960 | 4960 | 2480 |
| Reaktionsdauer | [h] | 12 | 12 | 12 | 12 | 12 |
| Temperatur | [°C] | 110 | 110 | 110 | 110 | 110 |
| Säurezahl | | 144 | 136 | 156 | 168 | 135 |
| Verseifungszahl | | 193 | | | | 163 |
| Tropfpunkt | 76 | | | | 84 | |
| Schmelzpunkt | 73.3 | | 75 | | | |
| Schmelzenthalpie | -198.5 | | -195 | | | |
| TGA | [Gew.-%, 300°/+30min] | 36,4/75 | | 45/87 | | |
| Iodzahl | [g$I_2$/100] | <1 | <1 | <1 | | 2 |

In den Figuren 1 bis 5 ist die Kettenlängenverteilung der Oxidate aus den Versuchen 1 bis 5 wiedergegeben

Herstellungsbeispiel 3: Oxidation von Reiskleiewachs unter heftigem Rühren

Ansatz:

| | |
|---|---|
| Reiskleiewachs (Typ 4) | 60 g |
| Chromschwefelsäure (100 g $CrO_3$/L) | 1060 g |

Durchführung:

In einem 2 l Reaktionsgefäß mit Rührer, Temperaturfühler, Tropftrichter und Rückflusskühler wird Chromschwefelsäure vorgelegt und auf 70 °C erwärmt. Anschließend wird geschmolzenes Reiskleiewachs portionsweise zugegeben. Die Temperatur des Reaktionsgemisches wird auf 110 °C eingestellt und 10 h mit 2000 U/min mit einer Dissolverscheibe gerührt. Das Heizen sowie Rühren wird eingestellt. Sobald sich die Phasen getrennt haben wird die wässrige Phase abgetrennt.

Die organische Phase wird mit einer wässrigen Lösung von Oxalsäure und Schwefelsäure sowie Wasser chromfrei gewaschen, in warme Zentrifugengläser abgelassen und zentrifugiert.

Herstellungsbeispiel 4: Oxidation von Reiskleiewachs unter Einsatz von Emulsionsvermittlern:

Ansatz:

| | |
|---|---|
| Reiskleiewachs | 500 g |
| Chromschwefelsäure (100 g $CrO_3$/L) | 2480 g |
| Emulsionsvermittler | 1 Gew.-% bezogen auf Reiskleiewachs |

Durchführung:

In einem 5 l Reaktionsgefäß mit Rührer, Temperaturfühler, Tropftrichter und Rückflusskühler wird Chromschwefelsäure vorgelegt und auf 70 °C erwärmt. Das Reiskleiewachs wird geschmolzen, mit dem entsprechenden Emulsionsvermittler vermischt und portionsweise zur Chromsäure zugegeben. Das Reaktionsgemisch wird bei 110 °C für 10 h gerührt. Das Heizen sowie Rühren wird eingestellt. Sobald sich die Phasen getrennt haben wird die wässrige Phase abgetrennt.

Die organische Phase wird mit einer wässrigen Lösung von Oxalsäure und Schwefelsäure sowie Wasser chromfrei gewaschen, in warme Zentrifugengläser abgelassen und zentrifugiert.

Tabelle 6: Eingesetzte Emulsionsvermittler

| | Bezeichnung | Summenformel |
|---|---|---|
| Emulsionsvermittler Typ 1 | Nonafluoro-1-Butansulfonsäure | $C_4HF_9SO_3$ |
| Emulsionsvermittler Typ 2 | Heptadecafluoro-1-Octansulfonsäure | $C_8HF_{17}SO_3$ |
| Emulsionsvermittler Typ 3 | Aluminiumtrichlorid | $AlCl_3$ |
| Emulsionsvermittler Typ 4 | Salzsäure 35 % | HCl |

Aus Tabelle 7 ist ersichtlich, dass eine einstufige Synthese von Reiskleiewachsoxidaten mit Säurezahlen größer 100 mgKOH/g unter Zuhilfenahme eines geeigneten Emulsionsvermittlers realisiert werden kann. Des Weiteren wird der Umsatz der Ester durch heftiges Rühren mit einer Dissolverscheibe erhöht.

Tabelle 7: Oxidation von Reiskleiewachs

| Versuchsnummer | | 6 | 7 | 8 | 9 | 10 | 11 |
|---|---|---|---|---|---|---|---|
| Reiswachs Typ 1 | [g] | | | | 500 | | |
| Reiswachs Typ 2 | [g] | 60 | 500 | | | 500 | |
| Reiswachs Typ 3 | [g] | | | | | | 500 |
| Reiswachs Typ 4 | | | | 500 | | | |
| Entharztes Rohmontanwachs | [g] | | | | | 500 | |
| Zusätze | | | | | | | |
| Chromschwefelsäure (100g $CrO_3$/l) | [ml] | 1060 | 4960 | 4960 | 4960 | 4960 | 4960 |
| Emulsionsvermittler Typ 1 | [g] | | 5 | | | | |
| Emulsionsvermittler Typ 2 | [g] | | | 5 | | | |
| Emulsionsvermittler Typ 3 | (g) | | | | 5 | | |
| Emulsionsvermittler Typ 4 | [g] | | | | | | |
| Bedingungen | | | | | | | |
| Reaktionsdauer | [h] | 12 | 12 | 12 | 12 | 12 | 12 |

(fortgesetzt)

| Zusätze | | | | | | | |
|---|---|---|---|---|---|---|---|
| Temperatur | [°C] | 110 | 110 | 110 | 110 | 110 | |
| Rührleistung | [U/min] | 2000 | 600 | 600 | 600 | 600 | 600 |
| Prüfergebnisse | | | | | | | |
| Säurezahl | [mgKOH/g] | 72 | 102 | 121 | 172 | 115 | 93 |
| Verseifungszahl | [mgKOH/g] | | 149 | | | | |
| Tropfpunkt | [°C] | 75.8 | | | | | |
| Schmelzpunkt | [°C] | 77.1 | 74.6 | | | | |
| Schmelzenthalpie | [J/g] | -202.4 | -195 | | | | |
| Iodzahl | [$gI_2$/100g] | | | | | 2 | |

Geeignete Bestimmungsmethoden zur Ermittlung der OH-Zahl sind z. B. DGF C-V 17 a (53), Ph. Eur. 2.5.3 Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 Liter Pyridin werden 50 ml Essigsäureanhydrid eingerührt) werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage, wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

| | |
|---|---|
| OHZ | = Hydroxylzahl in mg KOH/g Substanz |
| Va | = Verbrauch an Natronlauge in ml bei der Titration der Probe |
| Vb | = Verbrauch an Natronlauge in ml bei der Titration des Wirkwertes |
| c | = Stoffmengenkonzentration der Natronlauge in mol/l |
| t | = Titer der Natronlauge |
| M | = Molare Masse von KOH = 56,11 g/mol |
| E | = Probeneinwaage in g |

(Vb - Va) ist diejenige Menge der verwendeten Natronlauge in ml, die der bei der oben beschriebenen Acetylierung der zu messenden Probe gebundenen Menge an Essigsäure äquivalent ist.

B Wirkstoffe

DMAPA = 3-(Dimethylamino)propylamin

1. Rohstoffe

- Licowax S Flakes v. 01.07.2011 / Gersthofen
  = Montansäure (Wachssäuregemisch ca. $C_{24}$ - $C_{34}$)
  Spezifikation
  Analytik: SZ = 138 mgKOH/g → M = 406,6 g/mol 127 bis 160 mgKOH/g
  VZ = 169 mgKOH/g 157 bis 182 mgKOH/g
  Esterzahl = 31mgKOH/g
  Tropfpunkt = 79 bis 85 °C

- Licowax LP Flakes M 1000 v. 24.01.12 / Gersthofen
  Analytik: SZ = 122,2 mgKOH/g → M = 459,1 g/mol
  Spezifikation
  SZ= 113 bis 130 mgKOH/g
  VZ = 140 bis 170 mgKOH/g
  Tropfpunkt = 82 bis 89 °C

- RBW1 Säurezahl: 140 mg KOH/g
  (= oxidiertes Reiskleiewachs/Chromsäureoxidation von gereinigtem RBW Reiskleiewachs
  RBW2 Säurezahl: 148 mg KOH/g
  (= oxidiertes Reiskleiewachs/Chromsäureoxidation von rohem RBW Reiskleiewachs

Allgemeine Arbeitsvorschrift

Das Säureoxidat wird vorgelegt und bei etwa 80 °C aufgeschmolzen. Bei 80 bis 90 °C wird eine erste DMAPA-Teilmenge (120 Mol-%, bezogen auf das Säureoxidat) zudosiert. Der Ansatz wird erwärmt und am Rückfluss gehalten. Falls die Temperatur durch Bildung von Reaktion auf unter 150 °C fällt, wird langsam Destillat abgenommen, bis die Temperatur wieder auf 160 °C steigt. Wenn die Temperatur nicht mehr fällt, wird eine Nachreaktion bei voller Abnahme des Destillats für etwa 8 h durchgeführt. Die Temperatur wird auf 90 bis 100 °C abgekühlt. Die zweite Menge DMAPA (10 bis 20 Mol-%, bezogen auf eingesetztes Säureoxidat, abhängig von der Restsäurezahl) wird bei einer Temperatur von unter 100 °C zugegeben. Der Ansatz wird erneut auf 160 °C erhitzt und eine Nachreaktion bei 160 °C unter Normaldruck durchgeführt, bis ein Umsatz von mehr als 96 %, bezogen auf die Ausgangssäurezahl, erreicht ist. Dazu ist gegebenenfalls eine Nachdosierung von DMAPA und eine weitere Nachreaktion erforderlich. Zur Entfernung von überschüssigem DMAPA wird Stickstoff eingeleitet (etwa 1 L/h) und bei 160 °C volles Wasserstrahlvakuum angelegt, bis ein DMAPA-Gehalt von < 20 μg/g erreicht ist.

| Herstellungsbeispiel | Ausgangssäure | Einsatzmengen | | | Produkt | | |
|---|---|---|---|---|---|---|---|
| | | Säure in g | Gesamtmenge-DMAP A | | Rest-SZ in mgKOH/g | Umsetzungsgrad (ber. in m/m%) | Rest-DMAPA in µg/g |
| | | | in g | in mol-% bez. auf Säure | | | |
| 5 | Licowax S Flakes | 200,0 | 70,2 | 140 | 3,6 | 96,7 | 20 |
| 6 | Licowax S Flakes | 600,0 | 210,6 | 140 | 3,8 | 96,6 | 37 |
| 7 | Licowax LP Flakes | 630,0 | 196,0 | 140 | 2,7 | 97,4 | < 20 |
| 8 | Reiskleiewachs-oxidat RBW1 | 600,0 | 209,8 | 130 | 2,0 | 98,3 | 20 |
| 9 | Reiskleiewachs-oxidat RBW2 | 600,0 | 198,5 | 130 | 2,4 | 97,9 | 20 |

C Anwendungsbeispiele

Halbseitentest mit Haarnachbehandlungsmitteln zur Ermittlung der Konditionierungsleistung

Die Haare der Testperson werden mit 14 %iger Ethersulfatlösung (Genapol LRO; Sodium Laureth Sulfate, 28 %ig) gewaschen und mit einem Handtuch frottiert, anschließend werden sie in der Mitte gescheitelt und auf jeder Kopfhälfte 5 ml der Testsubstanz bzw. der Vergleichssubstanz aufgetragen und gut verteilt. Nach 1 min. Einwirkzeit werden die Haare ausgespült und anschließend im nassen und trockenen Zustand gekämmt. Die Konditionierleistung ergibt sich aus der Beurteilung von Kämmbarkeit und Sensorik (Geschmeidigkeit, Weichheit, Glätte).

| | | | |
|---|---|---|---|
| A | Testsubstanz | Konditionierungsmittel | 1.00 % |
| | Genapol® T-110 (Clariant) Ceteareth-11 | Emulgator | 1.50 % |
| | Cetyl Alkohol | Stabilisator | 3.00 % |
| | | | |
| B | Wasser | | ad 100 % |
| | Zitronensäure (25%) | Neutralisationsmittel | 1.00 % |
| | | | |
| C | Nipaguard® DMDMH (Clariant) DMDMH Hydantoin | Konservierungsmittel | 0.20 % |

Herstellung

I Die Komponenten von A mischen und bei ca. 75 °C aufschmelzen

II B auf ca. 75 °C erhitzen

III B unter Rühren zu A geben und kaltrühren

IV Bei ca. 30 °C die Komponente von C in die Mischung aus A und B einrühren

Resultat

| | |
|---|---|
| pH: | 3.8 bis 4.1 |
| Aussehen: | Creme |
| Viskosität (Brookfield, 20 °C, 20 Umd./Min): | ca. 3000 mPas |
| Stabilität: | 3 Monate bei RT und 40 °C |

Haarkonditionierleistung Anwendungsbeispiele:

| Nr. | Alkylamidopropyldimethylamine | SZ Fettsäure | C 16 | C 18 | C 20 | C 22 | C 24 | C 26 | C 28 | C 30 | C 32 | C 34 | C 36 | C 38 | M [g/mol] | Mp [°C] | Konditionierleistung |
|---|---|---|---|---|---|---|---|---|---|---|---|---|---|---|---|---|---|
| V1 | SPA | 197.8 | 1.5 | 98.3 | 0.2 | - | - | - | - | - | - | - | - | - | 283.6 | 72 | - |
| V2 | BAP | 180.6 | 0.8 | 45.3 | 4.7 | 47.3 | 1.9 | - | - | - | - | - | - | - | 310.6 | 70 | 0 |
| V3 | APA-22, | 167.6 | 0.7 | 4.0 | 6.6 | 86.2 | 2.5 | - | - | - | - | - | - | - | 334.7 | 85 | 0 |
| 1 | SPA + MAPDMA 9:1 | 191.2 | 1.3 | 88.5 | 0.2 | 0.3 | 0.9 | 1.9 | 2.4 | 2.5 | 1.5 | 0.5 | - | - | 293.4 | 67 | + |
| 2 | SPA + MAPDMA 7:3 | 177.9 | 1.1 | 68.8 | 0.1 | 0.9 | 2.7 | 5.7 | 7.2 | 7.5 | 4.5 | 1.5 | - | - | 315.3 | 59 | ++ |
| 3 | BAP + MAPDMA 9:1 | 175.7 | 0.7 | 40.8 | 4.2 | 42.9 | 2.6 | 1.9 | 2.4 | 2.5 | 1.5 | 0.5 | - | - | 319.3 | 66 | ++ |
| 4 | BAP + MAPDMA 7:3 | 165,8 | 0,6 | 31,7 | 3,3 | 34.0 | 4.0 | 5.7 | 7.2 | 7.5 | 4.5 | 1.5 | - | - | 338.4 | 60 | +++ |
| 5 | MAPDMA | 131.4 | - | - | - | 3 | 9 | 19 | 24 | 25 | 15 | 5 | - | - | 426.9 | 76 | +++ |
| 6 | SPA + RAPDMA 9:1 | 192.8 | 1.8 | 88.6 | 0.2 | 1.5 | 3.1 | 0.7 | 0.8 | 1.3 | 1.0 | 0.8 | 0.2 | - | 291.0 | 63- | 0 |
| 7 | SPA + RAPDMA 7:3 | 182.9 | 2.4 | 69.4 | 0.3 | 4.4 | 9.5 | 2.0 | 2.3 | 3.9 | 2.9 | 2.3 | 0.5 | 0.1 | 306.7 | -58 | ++ |
| 8 | BAP + RAPDMA 9:1 | 177.3 | 1.2 | 40.9 | 4.3 | 43.9 | 4.9 | 0.7 | 0.8 | 1.3 | 1.0 | 0.8 | 0.2 | - | 316.4 | -63 | + |
| 9 | BAP + RAPDMA 7:3 | 170.8 | 1.9 | 32.4 | 3,4 | 37.5 | 10.8 | 2.0 | 2.3 | 3.9 | 2.9 | 2.3 | 0.5 | 0.1 | 328.5 | -61 | +++ |
| 10 | RAPDMA | 148.0 | 4.5 | 2.2 | 0.4 | 14.6 | 31.7 | 6.6 | 7.5 | 13.1 | 9.8 | 7.8 | 1.5 | 0.3 | 379.1 | -68 | +++ |
| V4 | Vergleichsmuster (Marktstandard): Behenyltrimethylammoniumchloride | - | 0-2 | 0-7 | 11-16 | 75-81 | 0-2 | - | - | - | - | - | - | - | | | +++ |

M = mittlere Molmasse in [g/mol] der Fettsäure die zur Umsetzung zum Alkylamidopropyldimethylamine eingesetzt wird. Die Molmasse vom Alkylamidopropyldimethylamine ist jeweils 84 g/mol höher,
MAPDMA bedeutet Montanamidopropyldimethylamin, d.h. das Umsetzungsprodukt von Montanwachsoxidat mit DMAPA.
RAPDMA bedeutet Reisamidopropyldimethylamin, d.h. das Umsetzungsprodukt von Reiskleiewachsoxidat RBW1 mit DMAPA.

SPA (Genamin® SPA) basiert auf Stearinsäure, BAP Genamin® BAP auf einer Mischung aus Stearin- und Behensäure, APA-22 (Amidet® APA-22 von Kao) entspricht dem Stand der Technik (EP-A 1 586 298). Es basiert auf Behensäure.

Unter der Haarkonditionierleistung sind die Parameter "Kämmbarkeit" und "Haargefühl", also Begriffe wie "Geschmeidigkeit/Weichheit/Glätte" zu verstehen; sie wurde nach vorstehendem Test ermittelt. Die Bewertung ist dabei aufsteigend von 0 bis +++ (sehr gut).

Erfindungsgemäße Zubereitungen zeigen eine bessere Konditionierleistung oder eine vergleichbare Konditionierleistung, bei verbesserter Umweltverträglichkeit als die aus dem Stand der Technik (Vergleichsbeispiel 3 bzw. 4) bekannten.

Ein niedriger Schmelzpunkt bedeutet eine leichtere Verarbeitbarkeit. Bei der Verwendung von Mischungen mit kürzerkettigen Amidaminen (Beispiele 1 bis 4 und 6 bis 9) wird der Schmelzpunkt trotz ansteigender Molmasse erniedrigt.

Beispiele für kosmetische Zubereitungen mit erfindungsgemäßen Kombinationen

| Shampoo | S1 | S2 | S3 | S4 | S5 | S6 | S7 | S8 | S9 | S10 | S11 | S12 | S13 | S14 | S15 | S16 | S17 | S18 | S19 | S20 | S21 |
|---|---|---|---|---|---|---|---|---|---|---|---|---|---|---|---|---|---|---|---|---|---|
| Sodium Laureth Sulfate | 6 | 6 | 6 | 6 | 8 | 8 | 8 | 8 | 12 | 12 | 12 | 12 | 14 | 14 | 14 | 14 | 14 | 16 | 16 | 6 | 12 |
| Cocamidopropyl Betaine | 1 | 0 | 1 | 1,5 | 1,5 | 0 | 1,5 | 2,5 | 1,5 | 1,5 | 2 | 1,5 | 1,6 | 1 | 1 | 1 | 1 | 1,5 | 1 | 1,5 | 2 |
| Coconut Monoethanol-amide | 1,5 | 1,5 | 2,5 | 2,5 | 2,5 | 2,5 | 2,5 | 3 | 4 | 4 | 3 | 1,5 | 2 | 2,5 | 2,5 | 2,5 | 2,5 | 1,5 | 1,5 | 2,5 | 3 |
| CTAC | 2 | 2 | 1,7 | 1,7 | 1 | 1,2 | 1,5 | 1,7 | 2 | 1,7 | 1,5 | 1 | 2 | 1,7 | 1,5 | 1 | 1 | 1,5 | 1 | 1,7 | 1,5 |
| MAP DMA | 0,5 | 0,5 | 1 | 1,2 | 1 | 0,5 | 1 | 1,2 | 1 | 0,5 | 0,5 | 1,2 | 1 | 0,5 | 1 | 1 | 1,2 | 0,5 | 1,2 | 0 | 0 |
| SAP DMA | 1 | 0 | 0 | 0 | 1 | 0 | 2,3 | 0 | 1 | 1 | 0 | 0 | 1 | 0 | 2,3 | 0 | 0 | 0 | 2,8 | 0 | 0 |
| BAP DMA | 0 | 1 | 2,3 | 2,8 | 1 | 1 | 0 | 2,8 | 1 | 0 | 1 | 2,3 | 1 | 1 | 0 | 2,3 | 2,8 | 1 | 0 | 2,8 | 1 |
| RAP DMA | 0 | 0 | 0 | 0 | 0 | 0 | 0 | 0 | 0 | 0 | 0 | 0 | 0 | 0 | 0 | 0 | 0 | 0 | 0 | 1,2 | 0,5 |
| Laureth-7 | 3 | 3 | 3 | 3 | 2,5 | 2,5 | 2,5 | 2,5 | 2 | 2 | 2 | 2 | 2 | 2 | 2 | 2 | 2 | 1,5 | 1,5 | 3 | 2 |
| cationic guar | 0,5 | 0,5 | 0,5 | 1 | 1 | 1 | 1 | 1 | 1 | 1,5 | 1,5 | 1,5 | 2 | 2 | 2 | 2 | 2 | 2 | 2 | 1 | 1,5 |
| Polyquaternium-10 | 0,4 | 0,4 | 0,3 | 0,3 | 0,4 | 0,4 | 0,3 | 0,3 | 0,4 | 0,2 | 0,2 | 0,2 | 0,2 | 0,1 | 0,2 | 0,2 | 0,1 | 0,1 | 0,1 | 0,3 | 0,2 |
| Polyquaternium-7 | 0,4 | 0,4 | 0,2 | 0,2 | 0,4 | 0,4 | 0,2 | 0,2 | 0,4 | 0,2 | 0,1 | 0,1 | 0,1 | 0,1 | 0,1 | 0,1 | 0,1 | 0,1 | 0,1 | 0,2 | 0,1 |
| Carbomer | 0,05 | 0,05 | 0,05 | 0,2 | 0,2 | 0,2 | 0,2 | 0,2 | 0,2 | 0,3 | 0,3 | 0,4 | 0,3 | 0,3 | 0,45 | 0,45 | 0,45 | 0,45 | 0,45 | 0,2 | 0,3 |

| | | | | | | | | | | | | | | | | | | | | | |
|---|---|---|---|---|---|---|---|---|---|---|---|---|---|---|---|---|---|---|---|---|---|
| Cetearyl alcohol | 0,2 | 0,2 | 0,2 | 0,5 | 0,5 | 0,7 | 0,7 | 0,7 | 1 | 0,5 | 0,5 | 0,5 | 0,5 | 1 | 1 | 1 | 1 | 0,5 | 1 | 0,5 | 0,5 |
| Dimethicone | 0 | 0 | 0 | 0,5 | 0,5 | 1 | 1 | 1 | 1 | 1,5 | 1,5 | 1,7 | 1,5 | 1,5 | 2 | 2 | 2 | 2 | 2 | 0,5 | 1,5 |
| TMPPTS | 0 | 0 | 0,05 | 0,05 | 0,1 | 0,1 | 0,15 | 0,15 | 0,15 | 0,15 | 0,15 | 0,2 | 0,2 | 0,2 | 0,2 | 0,2 | 0,2 | 0,2 | 0,2 | 0,05 | 0,15 |
| Konservierung, Farbstoff, Parfüm | qs | qs | qs | qs | qs | qs | qs | qs | qs | qs | qs | qs | qs | qs | qs | qs | qs | qs | qs | qs | qs |
| Zitronen-/Milchsäure, NaOH, ad pH | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5,5-6,5 | 5.5-6.8 | 5.5-6.15 |
| aqua | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 |

Die beispielgemäßen Shampoos können eine Reihe optionaler Inhaltstoffe aufweisen, etwa bis zu 4 Gew.-% Anti-Schuppen-Wirkstoffe (z. B. Octopirox, Zinkpyrithion, Climbazol, oder deren Mischungen, jeweils bezogen auf den Aktivgehalt), bis zu 1 Gew.-% Faser-Reparatur-Wirkstoffe (z. B. Keratin, Trehalose, jeweils bezogen auf den Aktivgehalt), bis zu 5 Gew.-% Inhaltsstoffe, die das Erscheinungsbild der Zubereitung selbst verbessern (z. B. Glycoldistearat, EGDS, Glimmer, Zinnoxide, oder deren Mischungen, jeweils bezogen auf den Aktivgehalt).

| Conditioner | C1 | C2 | C3 | C4 | C5 | C6 | C7 | C8 | C9 | C10 | C11 | C12 | C13 | C14 | C15 | C16 |
|---|---|---|---|---|---|---|---|---|---|---|---|---|---|---|---|---|
| Stearamidopropyl Dimethylamine (TAS) | 0,50 | 0,50 | 0,50 | 0,50 | 0,50 | 1,00 | 1,00 | 1,00 | 1,00 | 1,00 | 1,50 | 1,50 | 1,50 | 1,50 | 1,50 | 1,00 |
| Cetrimonium chloride (CTAC) | 0,10 | 0,10 | 0,05 | 0,05 | 0,00 | 0,10 | 0,10 | 0,05 | 0,05 | 0,00 | 0,10 | 0,10 | 0,05 | 0,05 | 0,00 | 0,05 |
| Behentrimonium Chloride and DPG (BTAC) | 0,05 | 0,05 | 0,10 | 0,10 | 0,05 | 0,05 | 0,05 | 0,10 | 0,10 | 0,05 | 0,05 | 0,05 | 0,10 | 0,10 | 0,05 | 0,10 |
| Cetearyl alcohol | 2,00 | 2,00 | 2,00 | 2,00 | 4,00 | 4,00 | 4,00 | 4,00 | 4,00 | 6,00 | 6,00 | 6,00 | 6,00 | 8,00 | 8,00 | 4,00 |
| paraffin wax | 2,00 | 2,00 | 2,00 | 2,00 | 2,00 | 2,00 | 2,00 | 1,00 | 1,00 | 1,00 | 1,00 | 1,00 | 1,00 | 0,00 | 0,00 | 1,00 |
| MAP DMA | 1,50 | 1,50 | 1,50 | 1,50 | 1,50 | 1,00 | 1,00 | 1,00 | 1,00 | 1,00 | 0,50 | 0,50 | 0,50 | 0,50 | 0,50 | 0 |
| SAP DMA | 3,50 | 0,00 | 3,50 | 0,00 | 3,50 | 2,30 | 0,00 | 2,30 | 0,00 | 2,30 | 1,20 | 0,00 | 1,20 | 0,00 | 1,20 | 2,30 |
| BAP DMA | 0,00 | 3,50 | 0,00 | 3,50 | 0,00 | 0,00 | 2,30 | 0,00 | 2,30 | 0,00 | 0,00 | 1,20 | 0,00 | 1,20 | 0,00 | 0,00 |
| RAP DMA | 0 | 0 | 0 | 0 | 0 | 0 | 0 | 0 | 0 | 0 | 0 | 0 | 0 | 0 | 0 | 1 |
| Dimethicone | 2,50 | 2,50 | 2,50 | 2,50 | 2,50 | 3,00 | 3,00 | 3,00 | 2,00 | 2,00 | 2,00 | 2,00 | 1,50 | 1,50 | 1,00 | 3,00 |
| Dimethiconol | 2,50 | 2,50 | 2,00 | 2,00 | 1,50 | 1,00 | 1,00 | 1,00 | 1,00 | 1,00 | 2,00 | 1,00 | 2,00 | 1,50 | 1,00 | 1,00 |
| PEG-based polymer | 0,2 | 0,2 | 0,2 | 0,2 | 0,2 | 0,2 | 0,2 | 0,2 | 0,2 | 0,2 | 0,2 | 0,2 | 0,2 | 0,2 | 0,2 | 0.2 |
| Glycerin | 1,00 | 2,00 | 4,00 | 1,00 | 2,00 | 4,00 | 1,00 | 2,00 | 4,00 | 1,00 | 2,00 | 4,00 | 1,00 | 2,00 | 4,00 | 2,00 |
| Konservierung, Farbstoff, Parfüm | qs | qs | qs | qs | qs | qs | qs | qs | qs | qs | qs | qs | qs | qs | qs | qs |
| Zitronen- /Milchsäure, NaOH, | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5,0 | 3,5-5.0 |
| ad pH | | | | | | | | | | | | | | | | |
| aqua | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 | ad 100 |

Die beispielgemäßen Conditioner können eine Reihe optionaler Inhaltstoffe aufweisen, etwa bis zu 1 Gew.-% Anti-Schuppen-Wirkstoffe (z. B. Octopirox, Zinkpyrithion, Climbazol, oder deren Mischungen, jeweils bezogen auf den Aktivgehalt), bis zu 0,5 Gew.-% Faser-Reparatur-Wirkstoffe (z. B. Keratin, Trehalose, jeweils bezogen auf den Aktivgehalt), bis zu 2 Gew.-% Inhaltsstoffe, die Emotionen hervorrufen sollen (z. B. Kokos-, Argan- und/oder Jojobaöl; L-Lysin HCl, L-Arginin, L-Serin, L-Glutaminsäure; hydrolysiertes Soja-, Elastin- oder Weizenprotein, Keratin; Vitamin E und/oder A, Provitamin B5, jeweils bezogen auf den Aktivgehalt).

| Frisiercreme | K1 | K2 | K3 | K4 | K5 | K6 | K7 | K8 | K9 | K10 | K11 | K12 |
|---|---|---|---|---|---|---|---|---|---|---|---|---|
| Stearamidopropyl Dimethylamine | 0,50 | 0,50 | 0,50 | 1,00 | 1,00 | 1,00 | 1,50 | 1,50 | 1,50 | 1,50 | 1,00 | 1,50 |
| Behentrimonium Chloride | 0,30 | 0,30 | 0,30 | 0,30 | 0,30 | 0,30 | 0,30 | 0,30 | 0,30 | 0,30 | 0,30 | 0,30 |
| Cetearyl alcohol | 2,00 | 2,00 | 1,50 | 1,50 | 3,00 | 3,00 | 2,50 | 2,50 | 4,50 | 4,50 | 1,50 | 2,50 |
| MAP DMA | 1,50 | 1,50 | 1,00 | 1,00 | 0,70 | 0,70 | 0,50 | 0,50 | 0,50 | 0,50 | 0 | 0 |
| SAP DMA | 3,50 | 0,00 | 2,30 | 0,00 | 1,60 | 0,00 | 1,20 | 0,00 | 1,20 | 0,00 | 0,00 | 1,20 |
| BAP DMA | 0,00 | 3,50 | 0,00 | 2,30 | 0,00 | 1,60 | 0,00 | 1,20 | 0,00 | 1,20 | 2,30 | 0,00 |
| RAP DMA | 0 | 0 | 0 | 0 | 0 | 0 | 0 | 0 | 0 | 0 | 1 | 0,5 |
| Dimethicone/ dimethiconol | 2,50 | 2,50 | 3,00 | 1,50 | 1,50 | 1,00 | 1,00 | 0,50 | 0,50 | 0,50 | 1,50 | 1,00 |
| Glycerin | 0,50 | 1,50 | 1,00 | 1,00 | 2,50 | 2,50 | 2,00 | 2,00 | 2,50 | 2,50 | 1,00 | 2,00 |
| Petroleum jelly | 3,00 | 3,00 | 2,50 | 2,50 | 2,00 | 2,00 | 1,50 | 1,00 | 0,50 | 0,50 | 2,50 | 1,50 |
| Konservierung (z. B. DMDM Hydantoin) | 0,20 | 0,20 | 0,20 | 0,20 | 0,20 | 0,20 | 0,20 | 0,20 | 0,20 | 0,20 | 0,20 | 0,20 |
| Farbstoff, Parfüm | qs | qs | qs | qs | qs | qs | qs | qs | qs | qs | qs | qs |
| Zitronen- /Milchsäure, NaOH/$NH_4OH$, ad pH | 3,5-7,5 | 3,5-7,5 | 3,5-7,5 | 3,5-7,5 | 3,5-7,5 | 3,5-7,5 | 3,5-7,5 | 3,5-7,5 | 3,5-7,5 | 3,5-7,5 | 3,5-7,5 | 3,5-7,5 |
| aqua | ad 100 | ad 100 | ad 100 | ad 100 | ad 100 | ad 100 | ad 100 | ad 100 | ad 100 | ad 100 | ad 100 | ad 100 |

Die beispielgemäßen Frisiercremes können eine Reihe optionaler Inhaltstoffe aufweisen, etwa bis zu 0,15 Gew.-% Faser-Reparatur-Wirkstoffe (z. B. Natriumsulfat, Trehalose, jeweils bezogen auf den Aktivgehalt), bis zu 1 Gew.-% Inhaltsstoffe, die Emotionen hervorrufen sollen (z. B. Kokos-, Argan-, Mandel, Oliven und/oder Jojobaöl; L-Lysin HCl; hydrolysiertes Soja-, Elastin- oder Weizenprotein, Keratin; Vitamin E und/oder A, Provitamin B5, jeweils bezogen auf den Aktivgehalt), bis zu 0,5 Gew.-% Viskositätsmodifiziermittel (z. B. NaCl, KCl, Carbomere, Hydroxyethylcellulose, PEG-Polymere, oder deren Mischungen, jeweils bezogen auf den Aktivgehalt).

| Frisiermaske | M1 | M2 | M3 | M4 | M5 | M6 | M7 | M8 | M9 | M10 | M11 | M12 |
|---|---|---|---|---|---|---|---|---|---|---|---|---|
| Stearamidopropyl Dimethylamine (TAS) | 0,05 | 0,05 | 0,05 | 0,10 | 0,10 | 0,10 | 0,10 | 0,15 | 0,15 | 0,15 | 0,10 | 0,10 |
| Behentrimonium Chloride (BTAC) | 1,00 | 1,00 | 1,50 | 1,50 | 2,00 | 2,00 | 2,50 | 2,50 | 3,00 | 3,00 | 1,50 | 2,50 |
| Cetearyl alcohol | 7,00 | 7,00 | 6,00 | 6,00 | 5,50 | 5,50 | 5,00 | 5,00 | 4,00 | 4,00 | 6,00 | 5,00 |
| MAP DMA | 1,50 | 1,50 | 1,00 | 1,00 | 0,70 | 0,70 | 0,50 | 0,50 | 0,50 | 0,50 | 0 | 0 |
| SAP DMA | 3,50 | 0,00 | 2,30 | 0,00 | 1,60 | 0,00 | 1,20 | 0,00 | 1,20 | 0,00 | 0,00 | 1,20 |
| BAP DMA | 0,00 | 3,50 | 0,00 | 2,30 | 0,00 | 1,60 | 0,00 | 1,20 | 0,00 | 1,20 | 2,30 | 0,00 |
| RAP DMA | 0 | 0 | 0 | 0 | 0 | 0 | 0 | 0 | 0 | 0 | 1 | 0,5 |
| Dimethicone | 1,00 | 1,00 | 1,50 | 1,50 | 2,00 | 2,00 | 2,50 | 2,50 | 2,50 | 2,50 | 1,50 | 2,50 |
| Dimethiconol | 3,00 | 3,00 | 3,00 | 2,50 | 2,50 | 2,00 | 2,00 | 1,50 | 1,50 | 1,00 | 2,50 | 2,00 |
| glycerine/ glycerol | 1,00 | 1,00 | 1,00 | 1,00 | 2,00 | 2,00 | 2,00 | 1,50 | 1,50 | 1,50 | 1,00 | 2,00 |
| Petroleum jelly | 0,2 | 0,2 | 0,2 | 0,2 | 0,2 | 0,2 | 0,2 | 0,2 | 0,2 | 0,2 | 0.2 | 0.2 |
| Mineral oil | 0,30 | 0,30 | 0,30 | 0,20 | 0,20 | 0,15 | 0,10 | 0,10 | 0,10 | 0,00 | 0,20 | 0,10 |
| Konservierung (z. B. DMDM Hydantoin) | 0,20 | 0,20 | 0,20 | 0,20 | 0,20 | 0,20 | 0,20 | 0,20 | 0,20 | 0,20 | 0,20 | 0,20 |
| Farbstoff, Parfüm | qs | qs | qs | qs | qs | qs | qs | qs | qs | qs | qs | qs |
| Zitronen- /Milchsäure, NaOH/$NH_4$OH, ad pH | 3,5-5,5 | 3,5-5,5 | 3,5-5,5 | 3,5-5,5 | 3,5-5,5 | 3,5-5,5 | 3,5-5,5 | 3,5-5,5 | 3,5-5,5 | 3,5-5,5 | 3,5-5,5 | 3,5-5,5 |
| Aqua | ad 100 | ad 100 | ad 100 | ad 100 | ad 100 | ad 100 | ad 100 | ad 100 | ad 100 | ad 100 | ad 100 | ad 100 |

Die beispielgemäßen Frisiermasken können eine Reihe optionaler Inhaltstoffe aufweisen, etwa bis zu 0,2 Gew.-% Faser-Reparatur-Wirkstoffe (z. B. Natriumsulfat, Trehalose, jeweils bezogen auf den Aktivgehalt), bis zu 1 Gew.-% Inhaltsstoffe, die Emotionen hervorrufen sollen (z. B. Kokos-, Argan-, Mandel, Oliven und/oder Jojobaöl; L-Lysin HCl, L-Arginin, L-Serin, L-Glutaminsäure; hydrolysiertes Soja-, Elastin- oder Weizenprotein, Keratin; Vitamin E und/oder A, Provitamin B5, jeweils bezogen auf den Aktivgehalt), bis zu 0,2 Gew.-% Viskositätsmodifiziermittel (z. B. NaCl, KCl, Carbomere, Hydroxyethylcellulose, PEG-Polymere, oder deren Mischungen, jeweils bezogen auf den Aktivgehalt).

## Patentansprüche

**1.** Kombinationen aus kationischen Tensiden und/oder kationischen Polymeren mit Amidaminen der Formel (I),

$$R\text{-}C(O)\text{-}NH\text{-}(CH_2)_3\text{-}N(CH_3)_2 \qquad (I)$$

worin R-C(O) für die aliphatischen Säurereste eines Naturwachsoxidats steht.

**2.** Kombinationen aus kationischen Tensiden und/oder kationischen Polymeren mit

a) 10 bis 100 Gew.-% Amidaminen der Formel (I) und
b) 90 bis 0 Gew.-% Amidaminen der Formel (II),

$$R^1\text{-}C(O)\text{-}NH\text{-}(CH_2)_3\text{-}N(CH_3)_2 \qquad (II)$$

worin $R^1$-C(O) für mindestens einen ($C_{18}$-$C_{22}$)-aliphatischen Säurerest steht.

**3.** Kombinationen aus kationischen Tensiden und/oder kationischen Polymeren mit Amidaminen der Formel (Ia),

$$R^2\text{-}C(O)\text{-}NH\text{-}(CH_2)_3\text{-}N(CH_3)_2 \qquad (Ia)$$

worin $R^2$-C(O) für aliphatische Säurereste steht,
erhältlich durch Umsetzung von 3-(Dimethylamino)propylamin mit

a) 10 bis 100 Gew.-% eines Naturwachsoxidats und
b) 0 bis 90 Gew.-% mindestens einer ($C_{18}$-$C_{22}$)-aliphatischen Carbonsäure.

**4.** Kombinationen nach einem der Ansprüche 1 bis 3, wobei die Säurezahl des Naturwachsoxidats oder Mischung von Naturwachsoxidat und ($C_{18}$-$C_{22}$)-aliphatischen Carbonsäuren gemessen nach DIN EN ISO 2114 mindestens 70 mg KOH/g ist.

**5.** Kombinationen nach Anspruch 4, wobei die Säurezahl des Naturwachsoxidats im Bereich von 70 bis 200 mg KOH/g liegt.

**6.** Kombinationen nach einem der Ansprüche 1 bis 5, wobei das Naturwachsoxidat gewählt ist aus der Gruppe bestehend aus Reiskleiewachsoxidaten, Montanwachsoxidaten, Carnaubawachsoxidaten, Zuckerrohrwachsoxidaten, Sonnenblumenwachsoxidaten, Candellilawachsoxidaten und Jojobaöloxidaten.

**7.** Kombinationen nach einem der Ansprüche 6, wobei das Naturwachsoxidat gewählt ist aus Reiskleiewachsoxidaten und Montanwachsoxidaten.

**8.** Kombinationen nach einem der Ansprüche 1 bis 7, wobei in dem Naturwachs, aus dem das Naturwachsoxidat hergestellt wurde, mindestens 30 % Gew.-% der nativen Wachsester zu Carbonsäuren oxidiert wurde.

**9.** Kombinationen nach Anspruch 8, wobei mindestens 60 % der nativen Wachsester zu Carbonsäuren oxidiert wurden.

**10.** Kombinationen nach einem der Ansprüche 1 bis 9, wobei der Anteil der Amidamine mit einem Säurerest, der mindestens 24 C-Atome aufweist, mindestens 9,5 Gew.-% beträgt.

**11.** Kombinationen nach Anspruch 10, wobei der Anteil Anteil der Amidamine mit einem Säurerest, der mindestens 24

C-Atome aufweist, mindestens 25 Gew.-% beträgt.

**12.** Kombinationen nach Anspruch 10 oder 11, wobei die Säurereste 24 bis 40 C-Atome aufweisen.

**13.** Kosmetische Zubereitung enthaltend Kombinationen nach einem der vorangehenden Ansprüche.

**14.** Kosmetische Zubereitung nach einem der vorangehenden Ansprüche **dadurch gekennzeichnet, dass** zusätzlich wenigstens 0,01 Gew.-% Natriumchlorid enthalten sind.

**15.** Zubereitung nach einem der vorangehenden Ansprüche **dadurch gekennzeichnet, dass** wenigstens 0,3 Gew.-% an kationischen Tensiden enthalten sind, bezogen auf das Gesamtgewicht der Zubereitung.

**16.** Zubereitung nach einem der Ansprüche 13 bis 15 **dadurch gekennzeichnet, dass** wenigstens 0,1 Gew.-% an kationischen Polymeren enthalten sind, bezogen auf das Gesamtgewicht der Zubereitung.

**17.** Verwendung einer Zubereitung nach einem der Ansprüche 13 bis 16 als Haarpflegemittel.

**18.** Verwendung einer Zubereitung nach einem der Ansprüche 13 bis 16 als Haarspülung.

**19.** Verwendung einer Zubereitung nach einem der Ansprüche 13 bis 16 als Haarschampoo.

**20.** Kosmetische Zubereitung gemäß einem der Ansprüche 13 bis 16, enthaltend einen oder mehrere aliphatische Alkohole der Formel (III),

$$R^3\text{-OH} \qquad \text{(III)}$$

worin $R^3$ eine aliphatische gesättigte oder ungesättigte $C_8$-$C_{30}$-Kohlenwasserstoffgruppe ist, worin lineare aliphatische Kohlenwasserstoffgruppen 80 Gew.-% oder mehr ausmachen.

**21.** Kosmetische Zubereitung gemäß Anspruch 20, enthaltend wenigstens 0,3 Gew.-% an kationischen Tensiden und wenigstens 0,1 Gew.-% an kationischen Polymeren, bezogen auf das Gesamtgewicht der Zubereitung.

**22.** Kosmetische Zubereitung gemäß Anspruch 20 oder 21, enthaltend Cetyl- und/oder Stearylalkohol.

**23.** Kosmetische Zubereitung gemäß einem der Ansprüche 13 bis 22, enthaltend eine oder mehrere organische Säuren.

**24.** Kosmetische Zubereitung gemäß Anspruch 23, enthaltend Citronensäure, Milchsäure und/oder Glutaminsäure.

## Claims

**1.** A combination of cationic surfactants and/or cationic polymers with amide amines of the formula (I),

$$R\text{-C(O)-NH-}(CH_2)_3\text{-N}(CH_3)_2 \qquad \text{(I)}$$

in which R-C(O) represents the aliphatic acid radicals of a natural wax oxidate.

**2.** A combination of cationic surfactants and/or cationic polymers with

a) 10 to 100 wt% of amide amines of the formula (I) and
b) 90 to 0 wt% of amide amines of the formula (II),

$$R^1\text{-C(O)-NH-}(CH_2)_3\text{-N}(CH_3)_2 \qquad \text{(II)}$$

in which $R^1$-C(O) is at least one ($C_{18}$-$C_{22}$)-aliphatic acid radical.

**3.** A combination of cationic surfactants and/or cationic polymers with amide amines of the formula (Ia),

$R^2$-C(O)-NH-$(CH_2)_3$-$N(CH_3)_2$ (Ia)

in which $R^2$-C(O) represents aliphatic acid radicals,
obtainable by reaction of 3-(dimethylamino)propylamine with

a) 10 to 100 wt% of a natural wax oxidate and
b) 0 to 90 wt% of at least one ($C_{18}$-$C_{22}$)-aliphatic carboxylic acid.

**4.** The combination as claimed in any of claims 1 to 3, the acid number of the natural wax oxidate or mixture of natural wax oxidate and ($C_{18}$-$C_{22}$)-aliphatic carboxylic acids being at least 70 mg KOH/g as measured to DIN EN ISO 2114.

**5.** The combination as claimed in claim 4, the acid number of the natural wax oxidate being in the range from 70 to 200 mg KOH/g.

**6.** The combination as claimed in any of claims 1 to 5, the natural wax oxidate being selected from the group consisting of rice bran wax oxidates, montan wax oxidates, carnauba wax oxidates, sugarcane wax oxidates, sunflower wax oxidates, candelilla wax oxidates, and jojoba oil oxidates.

**7.** The combination as claimed in any of claims 6, the natural wax oxidate being selected from rice bran wax oxidates and montan wax oxidates.

**8.** The combination as claimed in any of claims 1 to 7, at least 30 wt% of the native wax esters in the natural wax from which the natural wax oxidate has been produced having been oxidized to carboxylic acids.

**9.** The combination as claimed in claim 8, at least 60% of the native wax esters having been oxidized to carboxylic acids.

**10.** The combination as claimed in any of claims 1 to 9, the fraction of the amide amines having an acid radical which has at least 24 C atoms being at least 9.5 wt%.

**11.** The combination as claimed in claim 10, the fraction fraction of the amide amines with an acid radical which has at least 24 C atoms being at least 25 wt%.

**12.** The combination as claimed in claim 10 or 11, the acid radicals having 24 to 40 C atoms.

**13.** A cosmetic preparation comprising combinations as claimed in any of the preceding claims.

**14.** The cosmetic preparation as claimed in any of the preceding claims, further comprising at least 0.01 wt% of sodium chloride.

**15.** The preparation as claimed in any of the preceding claims, comprising at least 0.3 wt% of cationic surfactants, based on the total weight of the preparation.

**16.** The preparation as claimed in any of claims 13 to 15, comprising at least 0.1 wt% of cationic polymers, based on the total weight of the preparation.

**17.** The use of a preparation as claimed in any of claims 13 to 16 as a hair care product.

**18.** The use of a preparation as claimed in any of claims 13 to 16 as a hair rinse.

**19.** The use of a preparation as claimed in any of claims 13 to 16 as a hair shampoo.

**20.** The cosmetic preparation as claimed in any of claims 13 to 16, comprising one or more aliphatic alcohols of the formula (III),

$R^3$-OH (III)

in which $R^3$ is an aliphatic saturated or unsaturated $C_6$-$C_{36}$-hydrocarbon group in which linear aliphatic hydrocarbon groups account for 80 wt% or more.

**21.** The cosmetic preparation as claimed in claim 20, comprising at least 0.3 wt% of cationic surfactants and at least 0.1 wt% of cationic polymers, based on the total weight of the preparation.

**22.** The cosmetic preparation as claimed in claim 20 or 21, comprising cetyl alcohol and/or stearyl alcohol.

**23.** The cosmetic preparation as claimed in any of claims 13 to 22, comprising one or more organic acids.

**24.** The cosmetic preparation as claimed in claim 23, comprising citric acid, lactic acid and/or glutamic acid.

**Revendications**

**1.** Combinaisons de tensioactifs cationiques et/ou de polymères cationiques avec des amidamines de formule (I) R-C(O)-NH-$(CH_2)_3$-$N(CH_3)_2$ (I) dans laquelle R-C(O) représente les radicaux acides aliphatiques d'un oxydat de cire naturelle.

**2.** Combinaisons de tensioactifs cationiques et/ou de polymères cationiques avec

a) 10 à 100 % en poids d'amidamines de formule (I) et
b) 90 à 0 % en poids d'amidamines de formule (II)

$$R^1\text{-C(O)-NH-}(CH_2)_3\text{-}N(CH_3)_2 \qquad \text{(II)}$$

dans laquelle $R^1$-C(O) représente au moins un radical acide aliphatique en ($C_{18}$-$C_{22}$).

**3.** Combinaisons de tensioactifs cationiques et/ou de polymères cationiques avec des amidamines de formule (Ia)

$$R^2\text{-C(O)-NH-}(CH_2)_3\text{-}N(CH_3)_2 \qquad \text{(Ia)}$$

dans laquelle $R^2$-C(O) représente des radicaux acides aliphatiques,
pouvant être obtenues par mise en réaction de 3-(diméthylamino)propylamine avec

a) 10 à 100 % en poids d'un oxydat de cire naturelle et
b) 0 à 90 % en poids d'au moins un acide carboxylique aliphatique en ($C_{18}$-$C_{22}$).

**4.** Combinaisons selon l'une quelconque des revendications 1 à 3, dans lesquelles l'indice d'acidité de l'oxydat de cire naturelle ou du mélange d'oxydat de cire naturelle et d'acides carboxyliques aliphatiques en ($C_{18}$-$C_{22}$) mesuré selon DIN EN ISO 2114 est d'au moins 70 mg KOH/g.

**5.** Combinaisons selon la revendication 4, dans lesquelles l'indice d'acidité de l'oxydat de cire naturelle se situe dans la plage allant de 70 à 200 mg KOH/g.

**6.** Combinaisons selon l'une quelconque des revendications 1 à 5, dans lesquelles l'oxydat de cire naturelle est choisi dans le groupe constitué par les oxydats de cire de son de riz, les oxydats de cire de lignite, les oxydats de cire de carnauba, les oxydats de cire de canne à sucre, les oxydats de cire de tournesol, les oxydats de cire de candelilla et les oxydats d'huile de jojoba.

**7.** Combinaisons selon l'une quelconque des revendications 6, dans laquelle l'oxydat de cire naturelle est choisi parmi les oxydats de cire de son de riz et les oxydats de cire de lignite.

**8.** Combinaisons selon l'une quelconque des revendications 1 à 7, dans lesquelles, dans la cire naturelle à partir de laquelle l'oxydat de cire naturelle a été fabriqué, au moins 30 % en poids des esters de cire natifs ont été oxydés en acides carboxyliques.

**9.** Combinaisons selon la revendication 8, dans lesquelles au moins 60 % des esters de cire natifs ont été oxydés en acides carboxyliques.

**10.** Combinaisons selon l'une quelconque des revendications 1 à 9, dans lesquelles la proportion des amidamines

contenant un radical acide qui comprend au moins 24 atomes C est d'au moins 9,5 % en poids.

**11.** Combinaisons selon la revendication 10, dans lesquelles la proportion proportion des amidamines contenant un radical acide qui comprend au moins 24 atomes C est d'au moins 25 % en poids.

**12.** Combinaisons selon la revendication 10 ou 11, dans lesquelles les radicaux acides comprennent 24 à 40 atomes C.

**13.** Préparation cosmétique contenant des combinaisons selon l'une quelconque des revendications précédentes.

**14.** Préparation cosmétique selon l'une quelconque des revendications précédentes, **caractérisée en ce qu'**elle contient en outre au moins 0,01 % en poids de chlorure de sodium.

**15.** Préparation selon l'une quelconque des revendications précédentes, **caractérisée en ce qu'**elle contient au moins 0,3 % en poids de tensioactifs cationiques, par rapport au poids total de la préparation.

**16.** Préparation selon l'une quelconque des revendications 13 à 15, **caractérisée en ce qu'**elle contient au moins 0,1 % en poids de polymères cationiques, par rapport au poids total de la préparation.

**17.** Utilisation d'une préparation selon l'une quelconque des revendications 13 à 16 en tant que produit pour le soin des cheveux.

**18.** Utilisation d'une préparation selon l'une quelconque des revendications 13 à 16 en tant qu'après-shampoing.

**19.** Utilisation d'une préparation selon l'une quelconque des revendications 13 à 16 en tant que shampoing pour les cheveux.

**20.** Préparation cosmétique selon l'une quelconque des revendications 13 à 16, contenant un ou plusieurs alcools aliphatiques de formule (III)

$$R^3\text{-OH} \qquad \text{(III)}$$

dans laquelle $R^3$ est un groupe hydrocarboné en $C_8$-$C_{30}$ aliphatique saturé ou insaturé, les groupes hydrocarbonés aliphatiques linéaires représentant 80 % en poids ou plus.

**21.** Préparation cosmétique selon la revendication 20, contenant au moins 0,3 % en poids de tensioactifs cationiques et au moins 0,1 % en poids de polymères cationiques, par rapport au poids total de la préparation.

**22.** Préparation cosmétique selon la revendication 20 ou 21, contenant de l'alcool cétylique et/ou stéarylique.

**23.** Préparation cosmétique selon l'une quelconque des revendications 13 à 22, contenant un ou plusieurs acides organiques.

**24.** Préparation cosmétique selon la revendication 23, contenant de l'acide citrique, de l'acide lactique et/ou de l'acide glutamique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02102334 A
- EP 1586298 A
- EP 1870080 A
- EP 1435363 A
- DE 10231886 A
- DE 10231886


### In der Beschreibung aufgeführte Nicht-Patentliteratur


- Ullmann's Encyclopedia of Industrial Chemistry. 2000
- **VLADIMIR VCELÁK.** Chemie und Technologie des Montanwachses. *Veredelung des Montanwachses,* 1959, 458 ff
- Ullmann's Encyclopedia of Industrial Chemistry. 1996, vol. A28, 110-122